# EUROPEAN PATENT APPLICATION

(11) **EP 4 741 491 A1**
(43) Date of publication of application: **13.05.2026**
(21) Application number: 24835249.4
(22) Date of filing: 24.06.2024
(51) Int. Cl.: C12N 1/21, C12N 15/70, C12P 13/08

(54) **ENGINEERING BACTERIUM FOR PRODUCING L-VALINE, CONSTRUCTION METHOD, AND USE**

(30) Priority: 04.07.2023 CN 202310809270; 04.07.2023 CN 202310809271; 04.07.2023 CN 202310809274; 04.07.2023 CN 202310809277; 04.07.2023 CN 202310809281
(71) Applicant: Heilongjiang Eppen Biotech Co., Ltd., Daqing, Heilongjiang 166200 (CN)
(72) Inventor: WEI, Aiying, Daqing, Heilongjiang 166200 (CN); MENG, Gang, Daqing, Heilongjiang 166200 (CN); ZHAO, Chunguang, Daqing, Heilongjiang 166200 (CN); ZHOU, Xiaoqun, Daqing, Heilongjiang 166200 (CN); ZHANG, Ying, Daqing, Heilongjiang 166200 (CN); BI, Guodong, Daqing, Heilongjiang 166200 (CN); SU, Houbo, Daqing, Heilongjiang 166200 (CN); YANG, Lipeng, Daqing, Heilongjiang 166200 (CN); ZHANG, Xiaoqin, Daqing, Heilongjiang 166200 (CN); WANG, Pan, Daqing, Heilongjiang 166200 (CN); WANG, Jiwei, Daqing, Heilongjiang 166200 (CN)
(74) Representative: Winter, Brandl - Partnerschaft mbB
(86) International application number: PCT/CN2024/100975
(87) International publication number: WO 2025/007767

(57) **Abstract**

The disclosure discloses an engineering bacterium producing L-valine-producing, a construction method thereof, and a use thereof. This disclosure finds that after knocking out the yjiT gene in Escherichia coli and expressing brnF and brnE, followed by knocking out the yjiV gene and expressing ilvE and ilvD, knocking out the trpR gene and expressing the ilvH mutant protein, knocking out the lacI and lacZ genes and expressing DNA polymerase, and/or, knocking out the ycgH gene and expressing ilvC, the resulting engineering bacterium can enhance L-valine production. The engineering bacterium of this disclosure can be used for producing L-valine and holds promising application prospects.

## Description

### CROSS REFERENCE TO THE RELATED APPLICATION

This application claims priority to five Chinese patent applications (with application numbers 2023108092706, 2023108092710, 2023108092744, 2023108092778, and 2023108092814 respectively), all filed on July 4, 2023. The entire contents of these five patent applications are hereby incorporated by reference into this document.

### TECHNICAL FIELD

The present disclosure relates to the field of biotechnology, specifically to an engineering bactierium for L-valine production, a construction method thereof, and use thereof.

### BACKGROUND

In microorganisms, L-valine (a type of branched-chain amino acid) is biosynthesized starting from pyruvate, proceeding via acetolactate, dihydroxyisovalerate, and ketoisovalerate. These intermediate metabolites are produced through reactions catalyzed by acetohydroxyacid synthase, acetohydroxyacid isomeroreductase, dihydroxyacid dehydratase, and transaminase B. However, these enzymes are also involved in the biosynthesis of L-isoleucine starting from 2-oxobutyrate and pyruvate, and L-leucine is biosynthesized starting from ketoisovalerate (an intermediate metabolite), proceeding via 2-isopropylmalate, 3-isopropylmalate, and ketoisocaproate. Therefore, because branched-chain amino acids (namely L-valine, L-isoleucine, and L-leucine) utilize the same enzymes for their biosynthesis processes, it is known to be challenging to industrially produce one type of branched-chain amino acid through fermentation. Additionally, there is a problem that industrial mass production is limited by feedback inhibition caused by L-valine (the end product) or its derivatives.

Escherichia coli, with its well-defined genetic background, is an attractive industrial production chassis for amino acid production. However, compared to Corynebacterium glutamicum, there are fewer reports on E. coli strains producing L-valine, possibly due to the more complex regulatory mechanisms of L-valine biosynthesis in E. coli. Acetohydroxyacid synthase (AHAS) is the rate-limiting enzyme in L-valine biosynthesis, and E. coli has three AHAS isozymes encoded by ilvBN, ilvGM, and ilvIH, with different properties and regulatory mechanisms. Park et al. reported the engineering of L-valine-producing strains through systematic metabolic engineering of Escherichia coli W3110 and Escherichia coli W, achieving a final L-valine yield of 60.7 g/L and a sugar-to-acid conversion rate of 0.22 g/g. In addition to mutagenesis breeding and conventional metabolic engineering modifications, cofactor balance is also considered a key bottleneck in improving L-valine yield. Since intracellular cofactors affect the metabolic network, signal transduction, and substance transport, thereby influencing the physiological functions of microbial cells. During the microbial fermentation production of chemicals, the potency and yield of chemicals are often limited by cofactor imbalance, primarily caused by the imbalanced expression of cofactor-dependent enzymes in the synthetic pathway. Savrasova and Stoynova et al. constructed an L-valine-producing engineering bacterium of E. coli MG1655 by replacing the native NADPH-dependent transaminase with a heterologous NADH-dependent leucine dehydrogenase. Under microaerobic conditions, the sugar-to-acid conversion rate (0.23 g/g) of this strain is only 35.4% of the maximum theoretical yield of 0.65 g/g. Developing high-throughput screening methods using biosensors, introducing exogenous cofactor regeneration pathways to balance intracellular cofactors, and constructing efficient industrial chassis production strains are key scientific issues that need to be addressed.

### SUMMARY

The technical problem to be solved by the present disclosure is how to improve the yield of L-valine.

In order to address the aforementioned technical problem, the present disclosure first provides an engineering bacterium for producing valine, which is a recombinant strain obtained by modifying a recipient bacterium; the modification comprises A1), A2), A3), A4), or A5);
A1) comprises A11), A12), and A13):
   A11) knocking out the yjiT gene of the recipient bacterium, inhibiting the expression of the yjiT gene, or inhibiting the activity of the protein encoded by the yjiT gene;
   A12) increasing the content or enhancing the activity of the protein encoded by the brnF gene in the recipient bacterium;
   A13) increasing the content or enhancing the activity of the protein encoded by the brnE gene in the recipient bacterium;
A2) comprises A21), A22), and A23);
   A21) knocking out the yjiV gene of the recipient bacterium, inhibiting the expression of the yjiV gene, or inhibiting the activity of the protein encoded by the yjiV gene;
   A22) increasing the content or enhancing the activity of the protein encoded by the ilvE gene in the recipient bacterium;
   A23) increasing the content or enhancing the activity of the protein encoded by the ilvD gene in the recipient bacterium;
A3) comprises A31) and A32):
   A31) knocking out the trpR gene of the recipient bacterium, inhibiting the expression of the trpR gene, or inhibiting the activity of the protein encoded by the trpR gene;
   A32) increasing the content or enhancing the activity of the protein encoded by the ilvH^{G14D , S17F} gene in the recipient bacterium; the ilvH^{G14D , S17F} gene is obtained by replacing the glycine codon at position 14 with an aspartate codon and the serine codon at position 17 with a phenylalanine codon in the ilvH gene;
A4) comprises A41), A42), and A43);
   A41) knocking out the lacI gene of the recipient bacterium, inhibiting the expression of the lacI gene, or inhibiting the activity of the protein encoded by the lacI gene;
   A42) knocking out the lacZ gene of the recipient bacterium, inhibiting the expression of the lacZ gene, or inhibiting the activity of the protein encoded by the lacZ gene;
   A43) increasing the content or enhancing the activity of DNA polymerase in the recipient bacterium;
A5) comprises A51) and A52):
   A51) knocking out the ycgH gene of the recipient bacterium, inhibiting the expression of the ycgH gene, or inhibiting the activity of the protein encoded by the ycgH gene;
   A52) increasing the content or enhancing the activity of the protein encoded by the ilvC gene in the recipient bacterium;

The recipient bacterium is Escherichia coli.

Specifically, the engineering bacterium for producing valine has the characteristics of B1), B2), B3), B4), or B5);
B1) comprises B11), B12), and B13):
   B11) the yjiT gene is not expressed or weakly expressed, or the activity of the protein encoded by the yjiT gene is reduced or lost;
   B12) the content of the protein encoded by the brnF gene is increased or the activity of the protein encoded by the brnF gene is enhanced;
   B13) the content of the protein encoded by the brnE gene is increased or the activity of the protein encoded by the brnE gene is enhanced;
B2) comprises B21), B22), and B23):
   B21) the yjiV gene is not expressed or weakly expressed, or the activity of the protein encoded by the yjiV gene is reduced or lost;
   B22) the content of the protein encoded by the ilvE gene is increased or the activity of the protein encoded by the ilvE gene is enhanced;
   B23) the content of the protein encoded by the ilvD gene is increased or the activity of the protein encoded by the ilvD gene is enhanced;
B3) comprises B31) and B32):
   B31) the trpR gene is not expressed or weakly expressed, or the activity of the protein encoded by the trpR gene is reduced or lost;
   B32) the content of the protein encoded by the ilvH^{G14D , S17F} gene is increased or the activity of the protein encoded by the ilvH^{G14D , S17F} gene is enhanced; the ilvH^{G14D , S17F} gene is obtained by replacing the glycine codon at position 14 with an aspartate codon and the serine codon at position 17 with a phenylalanine codon in the ilvH gene;
B4) comprises B41), B42), and B43):
   B41) the lacI gene is not expressed or weakly expressed, or the activity of the protein encoded by the lacI gene is reduced or lost;
   B42) the lacZ gene is not expressed or weakly expressed, or the activity of the protein encoded by the lacZ gene is reduced or lost;
   B43) the content of DNA polymerase is increased or the activity of DNA polymerase is enhanced;
B5) comprises B51) and B52):
   B51) the ycgH gene is not expressed or weakly expressed, or the activity of the protein encoded by the ycgH gene is reduced or lost;
   B52) the content of the protein encoded by the ilvC gene is increased or the activity of the protein encoded by the ilvC gene is enhanced;

The engineering bacterium is Escherichia coli.

Among them, the recipient bacterium contains the yjiT gene (GeneID: 945056, update date: 2023-04-14), the yjiV gene (GeneID: 2847669, update date: 2023-04-14), the trpR gene (GeneID: 948917, update date: 2023-04-14), the lacI gene (GeneID: 945007, update date: 2023-04-14), the lacZ gene (GeneID: 945006, update date: 2023-04-14), and the ycgH gene (GeneID: 2847703, update date: 2023-04-14).

In the aforementioned engineering bacterium, the brnF gene and the brnE gene can be sourced from Corynebacterium glutamicum.

Further, the brnF gene can encode the protein set forth in SEQ ID No. 2 in the sequence listing;
the brnE gene can encode the protein set forth in SEQ ID No. 3 in the sequence listing.

Even further, the brnF gene can be the DNA molecule shown at positions 832-1587 of SEQ ID No. 1 in the sequence listing;
the brnE gene can be the DNA molecule shown at positions 1584-1910 of SEQ ID No. 1 in the sequence listing.

In the aforementioned engineering bacterium, the ilvE gene can be sourced from Bacillus subtilis. The ilvD gene can be sourced from Escherichia coli.

Further, the ilvE gene can encode the protein set forth in SEQ ID No. 6 in the sequence listing. The ilvD gene can encode the protein set forth in SEQ ID No. 5 in the sequence listing.

Even further, the ilvE gene can be the DNA molecule shown at positions 808-1902 of SEQ ID No. 4 in the sequence listing. The ilvD gene can be the DNA molecule shown at positions 1977-3827 of SEQ ID No. 4 in the sequence listing.

In the aforementioned engineering bacterium, the ilvH gene can be sourced from Escherichia coli.

Further, the ilvH^{G14D , S17F} gene can encode the protein set forth in SEQ ID No. 8 in the sequence listing.

Even further, the ilvH^{G14D , S17F} gene can be the DNA molecule shown at positions 835-1326 of SEQ ID No. 7 in the sequence listing.

In the aforementioned engineering bacterium, the DNA polymerase can be sourced from Escherichia coli.

Further, the DNA polymerase can be the protein set forth in SEQ ID No. 10 in the sequence listing.

Even further, the encoding gene of the DNA polymerase can be the DNA molecule shown at positions 701-3352 of SEQ ID No. 9 in the sequence listing.

In the aforementioned engineering bacterium, the ilvC gene can be sourced from Escherichia coli.

Further, the ilvC gene can encode the protein set forth in SEQ ID No. 12 in the sequence listing.

Even further, the ilvC gene can be the DNA molecule shown at positions 794-2269 of SEQ ID No. 11 in the sequence listing.

The expression of the brnF gene, the brnE gene, the ilvE gene, the ilvD gene, the ilvH^{G14D , S17F} gene, the DNA polymerase encoding gene, and the ilvC gene in the engineering bacterium is initiated by promoters capable of initiating the expression of the corresponding genes in the engineering bacterium, including but not limited to strong promoters and constitutive promoters.

In one embodiment of the present disclosure, the expression of the brnF gene and the brnE gene is initiated by the Ptrc promoter, which is the DNA molecule shown at positions 758-831 of SEQ ID No. 1.

In one embodiment of the present disclosure, the expression of the ilvE gene is initiated by the Ptrc promoter, which is the DNA molecule shown at positions 1903-1976 of SEQ ID No. 4.

In another embodiment of the present disclosure, the expression of the ilvD gene is initiated by the PilvD promoter, which is the DNA molecule shown at positions 3828-3893 of SEQ ID No. 4.

In another embodiment of the present disclosure, the expression of the ilvH^{G14D , S17F} gene is initiated by the Ptrc promoter, which is the DNA molecule shown at positions 761-834 of SEQ ID No. 7.

In another embodiment of the present disclosure, the expression of the DNA polymerase encoding gene is initiated by the PxylF promoter, which is the DNA molecule shown at positions 3353-3617 of SEQ ID No. 9.

In another embodiment of the present disclosure, the expression of the ilvC gene is initiated by the Ptrc promoter, which is the DNA molecule shown at positions 2270-2343 of SEQ ID No. 11.

Specifically, A1) It can be achieved through gene editing using the CRISPR/Cas9 system, utilizing an sgRNA targeting the yjiT gene and a DNA fragment set forth in SEQ ID No. 1 as the donor.

A2) It can be achieved through gene editing using the CRISPR/Cas9 system, utilizing an sgRNA targeting the yjiV gene and a DNA fragment set forth in SEQ ID No. 4 as the donor.

A3) It can be achieved through gene editing using the CRISPR/Cas9 system, utilizing an sgRNA targeting the trpR gene and a DNA fragment set forth in SEQ ID No. 7 as the donor.

A4) It can be achieved through gene editing using the CRISPR/Cas9 system, utilizing sgRNAs targeting the lacI and lacZ genes and a DNA fragment set forth in SEQ ID No. 9 as the donor.

A5) It can be achieved through gene editing using the CRISPR/Cas9 system, utilizing an sgRNA targeting the ycgH gene and a DNA fragment set forth in SEQ ID No. 11 as the donor.

The recipient bacterium of the present disclosure comprises, but is not limited to, Escherichia coli. Any bacteria that comprise the yjiT gene, yjiV gene, trpR gene, lacI and lacZ genes, and ycgH gene and are capable of synthesizing L-valine can be used to prepare recombinant strains and produce L-lysine using the method of the present disclosure. Such bacteria may comprise Escherichia coli, Corynebacterium glutamicum, Brevibacterium lactofermentum, Corynebacterium pekinense, Brevibacterium ammoniagenes, Corynebacterium crenatum, Pantoea, Pantoea ananatis, Bacillus brevis, Brevibacterium lactofermentum, or Brevibacterium flavum. The yeast may be Saccharomyces cerevisiae or Pichia pastoris.

In one embodiment of the present disclosure, the Escherichia coli is Escherichia coli YP045 or Escherichia coli W3110.

In one embodiment of the present disclosure, the obtained recombinant strains are recombinant strains CGMCC22721-yjiT and W3110-yjiT. These recombinant strains are derived by knocking out a partial coding region of the yjiT gene on the genomes of the L-valine-producing strain CGMCC22721 and the wild-type Escherichia coli W3110, respectively, and simultaneously inserting the brnF-brnE genes (from Corynebacterium glutamicum ATCC13032) initiated by the Ptrc promoter, while keeping other nucleotides in their genomes unchanged.

In one embodiment of the present disclosure, the obtained recombinant strains are recombinant strains CGMCC22721-yjiV and W3110-yjiV. These recombinant strains are derived by knocking out the coding region of the yjiV gene on the genomes of the L-valine-producing strain CGMCC22721 and the wild-type Escherichia coli W3110, respectively, and simultaneously inserting the ilvE gene (from Bacillus subtilis subsp. subtilis str. 168) initiated by the Ptrc promoter and the ilvD gene (from Escherichia coli W3110) initiated by the PilvD promoter, while keeping other nucleotides in their genomes unchanged.

In one embodiment of the present disclosure, the obtained recombinant strains are recombinant strains CGMCC22721-trpR and W3110-trpR. These recombinant strains are derived by knocking out a partial coding region of the trpR gene on the genomes of the L-valine-producing strain CGMCC22721 and the wild-type Escherichia coli W3110, respectively, and simultaneously inserting the mutated ilvH^{G14D , S17F} genes initiated by the Ptrc promoter, while keeping other nucleotides in their genomes unchanged.

In one embodiment of the present disclosure, the obtained recombinant strains are recombinant strains CGMCC22721-lacIZ and W3110-lacIZ. These recombinant strains are derived by knocking out a partial coding region of the lacI-lacZ genes on the genomes of the L-valine-producing strain CGMCC22721 and the wild-type Escherichia coli W3110, respectively, and simultaneously inserting the DNA polymerase gene (from Escherichia coli BL21) initiated by the PxylF promoter, while keeping other nucleotides in their genomes unchanged.

In one embodiment of the present disclosure, the obtained recombinant strains are recombinant strains CGMCC22721-ycgH and W3110-ycgH. These recombinant strains are derived by knocking out a partial coding region of the ycgH gene on the genomes of the L-valine-producing strain CGMCC22721 and the wild-type Escherichia coli W3110, respectively, and simultaneously inserting the ilvC gene (from Escherichia coli W3110) initiated by the Ptrc promoter, while keeping other nucleotides in their genomes unchanged.

Among the aforementioned engineered bacteria, the modifications may also comprise any two, any three, any four, or all five of the above-mentioned A1), A2), A3), A4), and A5).

Specifically, the modifications can be: A1) and A2), A1) and A3), A1) and A4), A1) and A5), A2) and A3), A2) and A4), A2) and A5), A3) and A4), A3) and A5), A4) and A5), A1), A2), and A3), A1), A2), and A4), A1), A2), and A5), A1), A3), and A4), A1), A3), and A5), A1), A4), and A5), A2), A3), and A4), A2), A3), and A5), A2), A4), and A5), A3), A4), and A5), A1), A2), A3), and A4), A1), A2), A3), and A5), A1), A2), A4), and A5), A1), A3), A4), and A5), A2), A3), A4), and A5), and A1), A2), A3), A4), and A5).

Specifically, the engineered bacteria used for valine production also possess any two, any three, any four, or all five of the characteristics of the above-mentioned B1), B2), B3), B4), and B5).

In another embodiment of the present disclosure, the obtained recombinant strains are recombinant strains CGMCC22721-yjiT-yjiV and W3110-yjiT-yjiV. These recombinant strains are derived by knocking out a partial coding region of the yjiT gene on the genomes of the L-valine-producing strain CGMCC22721 and the wild-type Escherichia coli W3110, respectively, and inserting the brnF-brnE genes (from Corynebacterium glutamicum ATCC13032) initiated by the Ptrc promoter. Meanwhile, a partial coding region of the yjiV gene is knocked out, and the ilvE gene (from Bacillus subtilis subsp. subtilis str. 168) initiated by the Ptrc promoter and the ilvD gene (from Escherichia coli W3110) initiated by the PilvD promoter are inserted, while keeping other nucleotides in their genomes unchanged.

In another embodiment of the present disclosure, the obtained recombinant strains are recombinant strains CGMCC22721-yjiV-trpR and W3110-yjiV-trpR. These recombinant strains are derived by knocking out a partial coding region of the yjiV gene on the genomes of the L-valine-producing strain CGMCC22721 and the wild-type Escherichia coli W3110, respectively, and inserting the ilvE gene (from Bacillus subtilis subsp. subtilis str. 168) initiated by the Ptrc promoter and the ilvD gene (from Escherichia coli W3110) initiated by the PilvD promoter. Meanwhile, a partial coding region of the trpR gene is knocked out, and the mutated ilvH^{G14D , S17F} genes initiated by the Ptrc promoter are inserted, while keeping other nucleotides in their genomes unchanged.

In another embodiment of the present disclosure, the obtained recombinant strains are recombinant strains CGMCC22721-trpR-lacIZ and W3110-trpR-lacIZ. These recombinant strains are derived by knocking out a partial coding region of the trpR gene on the genomes of the L-valine-producing strain CGMCC22721 and the wild-type Escherichia coli W3110, respectively, and inserting the mutated ilvH^{G14D , S17F} genes initiated by the Ptrc promoter. Meanwhile, a partial coding region of the lacI-lacZ genes is knocked out, and the DNA polymerase gene (from Escherichia coli BL21) initiated by the PxylF promoter is inserted, while keeping other nucleotides in their genomes unchanged.

In another embodiment of the present disclosure, the obtained recombinant strains are recombinant strains CGMCC22721-lacIZ-ycgH and W3110-lacIZ-ycgH. These recombinant strains are derived by knocking out a partial coding region of the lacI-lacZ genes on the genomes of the L-valine-producing strain CGMCC22721 and the wild-type Escherichia coli W3110, respectively, and inserting the DNA polymerase gene (from Escherichia coli BL21) initiated by the PxylF promoter. Meanwhile, a partial coding region of the ycgH gene is knocked out, and the ilvC gene (from Escherichia coli W3110) initiated by the Ptrc promoter is inserted, while keeping other nucleotides in their genomes unchanged.

In another embodiment of the present disclosure, the obtained recombinant strains are recombinant strains CGMCC22721-yjiT-yjiV-trpR and W3110-yjiT-yjiV-trpR. These recombinant strains are derived by knocking out a partial coding region of the yjiT gene on the genomes of the L-valine-producing strain CGMCC22721 and the wild-type Escherichia coli W3110, respectively, and inserting the brnF-brnE genes (from Corynebacterium glutamicum ATCC13032) initiated by the Ptrc promoter. A partial coding region of the yjiV gene is knocked out, and the ilvE gene (from Bacillus subtilis subsp. subtilis str. 168) initiated by the Ptrc promoter and the ilvD gene (from Escherichia coli W3110) initiated by the PilvD promoter are inserted. Meanwhile, a partial coding region of the trpR gene is knocked out, and the mutated ilvH^{G14D , S17F} genes initiated by the Ptrc promoter are inserted, while keeping other nucleotides in their genomes unchanged.

In another embodiment of the present disclosure, the obtained recombinant strains are recombinant strains CGMCC22721-yjiV trpR-lacIZ and W3110-yjiV-trpR-lacIZ. These recombinant strains are derived by knocking out a partial coding region of the yjiV gene on the genomes of the L-valine-producing strain CGMCC22721 and the wild-type Escherichia coli W3110, respectively, and inserting the ilvE gene (from Bacillus subtilis subsp. subtilis str.168) initiated by the Ptrc promoter and the ilvD gene (from Escherichia coli W3110) initiated by the PilvD promoter. A partial coding region of the trpR gene is knocked out, and the mutated ilvH^{G14D , S17F} genes initiated by the Ptrc promoter are inserted. Meanwhile, a partial coding region of the lacI-lacZ genes is knocked out, and the DNA polymerase gene (from Escherichia coli BL21) initiated by the PxylF promoter is inserted, while keeping other nucleotides in their genomes unchanged.

In another embodiment of the present disclosure, the obtained recombinant strains are recombinant strains CGMCC22721-trpR-lacIZ-ycgH and W3110-trpR-lacIZ-ycgH. These recombinant strains are derived by knocking out a partial coding region of the trpR gene on the genomes of the L-valine-producing strain CGMCC22721 and the wild-type Escherichia coli W3110, respectively, and inserting the mutated ilvH^{G14D , S17F} genes initiated by the Ptrc promoter. A partial coding region of the lacI-lacZ genes is knocked out, and the DNA polymerase gene (from Escherichia coli BL21) initiated by the PxylF promoter is inserted. Meanwhile, a partial coding region of the ycgH gene is knocked out, and the ilvC gene (from Escherichia coli W3110) initiated by the Ptrc promoter is inserted, while keeping other nucleotides in their genomes unchanged.

In another embodiment of the present disclosure, the obtained recombinant strains are recombinant strains CGMCC22721-yjiT-yjiV-trpR-lacIZ and W3110-yjiT-yjiV-trpR-lacIZ. These recombinant strains are derived by knocking out a partial coding region of the yjiT gene on the genomes of the L-valine-producing strain CGMCC22721 and the wild-type Escherichia coli W3110, respectively, and inserting the brnF-brnE genes (from Corynebacterium glutamicum ATCC13032) initiated by the Ptrc promoter. A partial coding region of the yjiV gene is knocked out, and the ilvE gene (from Bacillus subtilis subsp. subtilis str.168) initiated by the Ptrc promoter and the ilvD gene (from Escherichia coli W3110) initiated by the PilvD promoter are inserted. A partial coding region of the trpR gene is knocked out, and the mutated ilvH^{G14D , S17F} genes initiated by the Ptrc promoter are inserted. Meanwhile, a partial coding region of the lacI-lacZ genes is knocked out, and the DNA polymerase gene (from Escherichia coli BL21) initiated by the PxylF promoter is inserted, while keeping other nucleotides in their genomes unchanged.

In another embodiment of the present disclosure, the obtained recombinant strains are recombinant strains CGMCC22721-yjiV-trpR-lacIZ-ycgH and W3110-yjiV-trpR-lacIZ-ycgH. These recombinant strains are derived by knocking out a partial coding region of the yjiV gene on the genomes of the L-valine-producing strain CGMCC22721 and the wild-type Escherichia coli W3110, respectively, and inserting the ilvE gene (from Bacillus subtilis subsp. subtilis str.168) initiated by the Ptrc promoter and the ilvD gene (from Escherichia coli W3110) initiated by the PilvD promoter. A partial coding region of the trpR gene is knocked out, and the mutated ilvH^{G14D , S17F} genes initiated by the Ptrc promoter are inserted. A partial coding region of the lacI-lacZ genes is knocked out, and the DNA polymerase gene (from Escherichia coli BL21) initiated by the PxylF promoter is inserted. Meanwhile, a partial coding region of the ycgH gene is knocked out, and the ilvC gene (from Escherichia coli W3110) initiated by the Ptrc promoter is inserted, while keeping other nucleotides in their genomes unchanged.

In another embodiment of the present disclosure, the obtained recombinant strains are recombinant strains CGMCC22721-yjiT-yjiV-trpR-lacIZ-ycgH and W3110-yjiT-yjiV-trpR-lacIZ-ycgH.These recombinant strains CGMCC22721-yjiT-yjiV-trpR-lacIZ-ycgH and W3110-yjiT-yjiV-trpR-lacIZ-ycgH are respectively derived by knocking out a partial coding region of the yjiT gene on the genomes of the L-valine-producing strain CGMCC22721 and the wild-type Escherichia coli W3110, and inserting the brnF-brnE genes (from Corynebacterium glutamicum ATCC13032) initiated by the Ptrc promoter. A partial coding region of the yjiV gene is knocked out, and the ilvE gene (from Bacillus subtilis subsp. subtilis str.168) initiated by the Ptrc promoter and the ilvD gene (from Escherichia coli W3110) initiated by the PilvD promoter are inserted. A partial coding region of the trpR gene is knocked out, and the mutated ilvH^{G14D , S17F} genes initiated by the Ptrc promoter are inserted. A partial coding region of the lacI-lacZ genes is knocked out, and the DNA polymerase gene (from Escherichia coli BL21) initiated by the PxylF promoter is inserted. Meanwhile, a partial coding region of the ycgH gene is knocked out, and the ilvC gene (from Escherichia coli W3110) initiated by the Ptrc promoter is inserted, while keeping other nucleotides in their genomes unchanged.

The present disclosure also provides a method for preparing an engineering bacterium for valine production, the method comprising: modifying a recipient bacterium according to the above A1), A2), A3), A4), or A5) to obtain the target engineering bacterium; the recipient bacterium is Escherichia coli.

The above method may also comprise modifying the recipient bacterium with any two, any three, any four, or all five of the above-mentioned five modifications A1), A2), A3), A4), and A5).

The present disclosure also provides a method for preparing L-valine, the method comprising: culturing the engineering bacterium to obtain L-valine.

In the above method, the engineering bacterium can be cultured using a medium that enables the growth of the engineering bacterium;
and/or, the recombinant engineering bacterium is cultured under conditions that enable the growth of the engineering bacterium.

The present disclosure also provides a product for producing L-valine, the product being any one of P1), P2), P3), P4), and P5) or a product composed of any two, any three, any four, or all five of these five:
P1) is a substance that achieves A11), A12), and A13) separately or simultaneously:
   A11) knocking out the yjiT gene of the recipient bacterium or inhibiting the expression of the yjiT gene or inhibiting the activity of the protein encoded by the yjiT gene;
   A12) increasing the content or enhancing the activity of the protein encoded by the brnF gene in the recipient bacterium;
   A13) increasing the content or enhancing the activity of the protein encoded by the brnE gene in the recipient bacterium;
P2) is a substance that achieves A21), A22), and A23) separately or simultaneously:
   A21) knocking out the yjiV gene of the recipient bacterium or inhibiting the expression of the yjiV gene or inhibiting the activity of the protein encoded by the yjiV gene;
   A22) increasing the content or enhancing the activity of the protein encoded by the ilvE gene in the recipient bacterium;
   A23) increasing the content or enhancing the activity of the protein encoded by the ilvD gene in the recipient bacterium;
P3) is a substance that achieves A31) and A32) separately or simultaneously:
   A31) knocking out the trpR gene of the recipient bacterium or inhibiting the expression of the trpR gene or inhibiting the activity of the protein encoded by the trpR gene;
   A32) increasing the content or enhancing the activity of the proteins encoded by the ilvH^{G14D , S17F} genes in the recipient bacterium;
P4) is a substance that achieves A41), A42), and A43) separately or simultaneously:
   A41) knocking out the lacI gene of the recipient bacterium or inhibiting the expression of the lacI gene or inhibiting the activity of the protein encoded by the lacI gene;
   A42) knocking out the lacZ gene of the recipient bacterium or inhibiting the expression of the lacZ gene or inhibiting the activity of the protein encoded by the lacZ gene;
   A43) increasing the content or enhancing the activity of DNA polymerase in the recipient bacterium;
P5) is a substance that achieves A51) and A52) separately or simultaneously:
   A51) knocking out the ycgH gene of the recipient bacterium or inhibiting the expression of the ycgH gene or inhibiting the activity of the protein encoded by the ycgH gene;
   A52) increasing the content or enhancing the activity of the protein encoded by the ilvC gene in the recipient bacterium.

The use of the engineering bacterium or the product in the production of L-valine, or in the preparation of products for producing L-valine, or in the preparation of foods, feeds, or pharmaceuticals containing L-valine, also falls within the scope of protection of the present disclosure.

The engineering bacterium, its preparation method, and related products of the present disclosure can be used to produce various products, including but not limited to valine in the examples. The produced products can also be glutamic acid, threonine, tryptophan, arginine, lysine, glycine, alanine, leucine, isoleucine, methionine, proline, serine, tyrosine, cysteine, phenylalanine, asparagine, glutamine, aspartic acid, histidine, shikimic acid, protocatechuic acid, succinic acid, α-ketoglutaric acid, citric acid, ornithine, citrulline, etc.

The present disclosure will be further described in detail below in conjunction with specific embodiments. The provided examples are only for illustrating the present disclosure and not for limiting its scope. The examples provided below can serve as a guide for further improvement by ordinary technicians in this technical field and do not constitute any limitation on the present disclosure in any way.

### Deposit Description

Taxonomic designation: Escherichia coli.
Strain number: YP045.
Name of the depositing institution: General Microbiological Center, China Committee for Culture Collection of Microorganisms.
Abbreviation of the depositing institution: CGMCC.
Address of the depositing institution: No. 3, Courtyard 1, Beichen West Road, Chaoyang District, Beijing, Postal Code: 100101.
Date of deposit: June 15, 2021.
Registration number at the deposit center: CGMCC No. 22721.

### BRIEF DESCRIPTION OF THE DRAWINGS

FIG. 1 shows a formula of fermentation medium (the remainder is water).
FIG. 2 shows a fermentation control process.
FIG. 3 shows yield and significance analysis of L-valine.

### DETAILED DESCRIPTION

Unless otherwise specified, the experimental methods in the following examples are conventional methods, carried out in accordance with the techniques or conditions described in the literature within the field or according to the product instructions. Unless otherwise specified, the materials, reagents, instruments, etc., used in the following examples can be obtained through commercial channels. For quantitative experiments in the following examples, three replicate experiments are set up, and the results are taken as the average value. Unless otherwise specified, in the following examples, the first position of each nucleotide sequence in the sequence listing is the 5' end nucleotide of the corresponding DNA/RNA, and the last position is the 3' end nucleotide of the corresponding DNA/RNA.

In the following examples, the L-valine-producing strain CGMCC22721 is Escherichia coli YP045. This strain is deposited at the General Microbiological Center, China Committee for Culture Collection of Microorganisms on June 15, 2021, with the deposit number CGMCC No.22721. Ningxia Yipin Biotechnology Co., Ltd., the depositor of Escherichia coli YP045, has authorized Heilongjiang Yipin Biotechnology Co., Ltd. to use this strain.

### Example 1: Construction of an engineering bacterium with the yjiT gene deleted and the brnF-brnE genes overexpressed on the genome

Based on the genome sequence of Escherichia coli W3110 published by NCBI, the CRISPR/Cas9 gene-editing technology is used to knock out the yjiT gene in the L-valine-producing strain CGMCC22721 and the wild-type Escherichia coli W3110 (it is confirmed by sequencing that these strains retained a complete yjiT gene (GeneID: 945056, updated on April 14, 2023) on their chromosomes). Meanwhile, the brnF-brnE gene (branched-chain amino acid exporter, a branched-chain amino acid export protein) initiated by the Ptrc promoter, sourced from Corynebacterium glutamicum ATCC13032, is inserted. This is done to further investigate in depth the impact of these genes on L-valine synthesis.

### I. Construction of sgRNA

Based on the genome sequence of Escherichia coli W3110 published by NCBI, the CRISPR RGEN Tools (http://www.rgenome.net/cas-designer/) is used to design the target sequence of sgRNA. After selecting an appropriate target sequence of sgRNA, the homologous arm sequences of the linearized pGRB cloning vector are added at the 5' and 3' ends of the target sequence to form a complete sgRNA plasmid through recombination.

The sgRNA fragment is amplified without a template, only requiring a PCR annealing process. The system and program are as follows: PCR reaction system comprising sgRNA-1F 10 µL, sgRNA-1R 10 µL; PCR reaction program: denaturation at 95°C for 5 min, annealing at 50°C for 1 min. After annealing, the target DNA fragment of sgRNA is recovered using a DNA purification kit, its DNA concentration is measured, and the concentration is diluted to 100 ng/µL.

The pGRB plasmid is digested with SpeI and then dephosphorylated. The Gibson Assembly kit (New England Biolabs) is used for the recombination of sgRNA and the dephosphorylated linearized plasmid. Recombination system comprises: NEB assembly enzyme 2.5 µL, dephosphorylated linearized plasmid 2 µL, target DNA fragment of sgRNA 0.5 µL. After assembly at 50°C for 30 min, the product is transformed into DH5α competent cells. The plasmid is extracted and identified by sequencing using the sequencing primers sgRNA-PF/sgRNA-PR. The plasmid with correct sequencing results is named pGRB-sgRNA-1.

The primers used in this experiment are synthesized by Invitrogen (Shanghai). The nucleotides with underlines are the homologous arm sequences of the pGRB cloning vector, and the nucleotides in bold are the sgRNA sequences: 
sgRNA-1F:5'-TGACAGCTAGCTCAGTCCTAGGTATAATACTAGTGTACGCGTTGCCAAT
TCT ATGTTTTAGAGCTAGAAATAGCAAGTTAAAATAAGG-3' (SEQ ID No.14)
 
sgRNA-1R:5'-CCTTATTTTAACTTGCTATTTCTAGCTCTAAAACATAGAATTGGCAACG
CGT ACACTAGTATTATACCTAGGACTGAGCTAGCTGTCA-3' (SEQ ID No.15)
sgRNA-PF: 5'- GTCTCATGAGCGGATACATATTTG-3' (SEQ ID No.16)
sgRNA-PR: 5'- GCGTCAGGTGCATAAACAGA-3' (SEQ ID No.17)

### II. PCR amplification of homologous recombination fragments

Based on the genome sequence of Escherichia coli W3110 published by NCBI, primers for knocking out the yjiT gene and primers for inserting the Ptrc-brnF-brnE gene sequence are designed and synthesized. The yjiT gene in the L-valine-producing strain CGMCC22721 and the wild-type Escherichia coli W3110 is knocked out, and the brnF-brnE gene initiated by the Ptrc promoter (sourced from Corynebacterium glutamicum ATCC13032) is inserted using the CRISPR/Cas9 gene-editing method.

The primers are synthesized by Invitrogen (Shanghai):
P1: 5'- GAGTGATGAGCGGTTGAAG-3' (SEQ ID No.18)
P2: 
5'-CTCACAATTCCACACATTATACGAGCCGGATGATTAATTGTCAAAAAACAGGCAGCAA
AGTCC-3' (SEQ ID No.19)
 
P3:5'-CGTATAATGTGTGGAATTGTGAGCGGATAACAATTTCACACAGGAAACAGACC
GTGCAAAAAACGCAAGAG-3' (SEQ ID No.20)
P4: 5'- CTTCACAGGTAGTGCTTTTAGTTAGAAAAGATTCACCAGTCCAAC-3' (SEQ ID No.21)
P5: 5'- GTGAATCTTTTCTAACTAAAAGCACTACCTGTGAAGG-3' (SEQ ID No.22)
P6: 5'- CTGCGGCAATAATCAACG-3' (SEQ ID No.23)

Using the genomic DNA of Escherichia coli W3110 as a template, PCR amplification is carried out with primers P1/P2, P5/P6, and KAPA HiFi HotStart (Shanghai Huayishichuang Biotechnology Co., Ltd., KK2601) to obtain upper and lower homologous arm fragments with sizes of 801 bp and 684 bp, respectively. Using the genomic DNA of Corynebacterium glutamicum ATCC13032 as a template, PCR amplification is carried out with primers P3/P4 and KAPA HiFi HotStart to obtain a Ptrc-brnF-brnE gene fragment with a size of 1153 bp. After the PCR reaction, agarose gel electrophoresis recovery is carried out using a column-based DNA gel recovery kit. The recovered DNA is subjected to overlap PCR with primers P1/P6 to obtain the homologous recombination DNA fragment ΔyjiT-Ptrc-brnF-brnE (SEQ ID No.1) with a size of 2579 bp.

In SEQ ID No.1, positions 758-831 represent the Ptrc promoter, positions 832-1910 represent the brnF-brnE gene, which encodes the brnF protein shown in SEQ ID No.2 (the coding sequence is positions 832-1587 of SEQ ID No.1) and the brnE protein shown in SEQ ID No.3 (the coding sequence is positions 1584-1910 of SEQ ID No.1).

### III. Preparation of competent cells and transformation

The pREDCas9 plasmid (containing a spectinomycin resistance gene) is extracted and transformed into the competent cells of the L-valine-producing strain CGMCC22721 and Escherichia coli W3110, respectively. The cells are spread on 2-YT agar plates containing spectinomycin (100 mg/L) and cultured at 32°C. Single colonies resistant to spectinomycin (100 mg/L) are selected and identified by PCR using primers pRedCas9-PF/pRedCas9-PR. The transformants containing the pREDCas9 plasmid, namely CGMCC22721-Cas9 and W3110-Cas9, with a fragment size of 943 bp are obtained.

The primers are synthesized by Invitrogen (Shanghai):
pRedCas9-PF: 5'- GCAGTGGCGGTTTTCATG-3' (SEQ ID No.24)
pRedCas9-PR: 5'- CCTTGGTGATCTCGCCTTTC-3' (SEQ ID No.25)

Competent cells of CGMCC22721-Cas9 and W3110-Cas9 are prepared. When the cell density reached OD₆₀₀ = 0.1, IPTG with a final concentration of 0.1 mM is added to induce λ-Red-mediated homologous recombination. When OD₆₀₀ = 0.4, the cells are collected to prepare competent cells, which are then transformed with the pGRB-sgRNA-1 plasmid and the homologous recombination DNA fragment △yjiT-Ptrc-brnF-brnE, respectively. The cells are spread on 2-YT agar plates containing spectinomycin (100 mg/L) and ampicillin (100 mg/L) and cultured at 32°C for 12 h. After subculturing the single colonies obtained from the culture, PCR identification is carried out using primers P7/P8. Positive transformants with a PCR-amplified fragment size of 1720 bp are obtained.

The primers are synthesized by Invitrogen (Shanghai):
P7: 5'- GCTGTATTCCTTATGTGGACC-3' (SEQ ID No.26)
P8: 5'- GCAGGAATCCAAAGTCAGC-3' (SEQ ID No.27)

The positive transformants are inoculated into 2-YT medium containing spectinomycin (100 mg/L) and arabinose with a final concentration of 0.2% to eliminate the plasmid pGRB-sgRNA-1. Colonies that grow on spectinomycin (100 mg/L) but not on ampicillin (100 mg/L) are selected. These colonies are then transferred to 2-YT medium and cultured at 42°C to eliminate the pREDCas9 plasmid. Colonies that do not grow on spectinomycin (100 mg/L) but grow on antibiotic-free 2-YT are selected. PCR identification is carried out again using primers P1/P6. Positive strains with a PCR-amplified fragment size of 2579 bp (SEQ ID No.1) are obtained. The positive strains are sent for sequencing, and the strains with correct sequencing results are named CGMCC22721-yjiT and W3110-yjiT, respectively.

The recombinant strains CGMCC22721-yjiT and W3110-yjiT both lack the yjiT gene and overexpress the brnF-brnE gene initiated by the Ptrc promoter (sourced from Corynebacterium glutamicum ATCC13032). Specifically, the recombinant strains CGMCC22721-yjiT and W3110-yjiT are obtained by knocking out part of the coding region of the yjiT gene on the genomes of the L-valine-producing strain CGMCC22721 and the wild-type Escherichia coli W3110, respectively, and inserting the brnF-brnE gene initiated by the Ptrc promoter (sourced from Corynebacterium glutamicum ATCC13032), while keeping other nucleotides in their genomes unchanged.

### Example 2: Construction of an engineering bacterium with the yjiV gene deleted and the ilvE-ilvD genes overexpressed in the genome.

Based on the genome sequence of Escherichia coli W3110 published by NCBI, the CRISPR/Cas9 gene editing technology is used to knock out the yjiV gene in the L-valine-producing strain CGMCC22721 and the wild-type Escherichia coli W3110 (it is confirmed by sequencing that these strains retained the complete yjiV gene (GeneID: 2847669, updated on April 14, 2023) on their chromosomes). Meanwhile, the ilvE gene (branched-chain amino-acid aminotransferase, from Bacillus subtilis subsp. subtilis str. 168) under the control of the Ptrc promoter and the ilvD gene (dihydroxyacid dehydratase, from Escherichia coli W3110) under the control of the PilvD promoter are inserted. This is done to further investigate the impact of these genes on L-valine synthesis.

### I. Construction of sgRNA

Based on the genome sequence of Escherichia coli W3110 published by NCBI, the sgRNA target sequence is designed using CRISPR RGEN Tools (http://www.rgenome.net/cas-designer/). After selecting an appropriate sgRNA target sequence, the homologous arm sequences of the linearized pGRB cloning vector are added at the 5' and 3' ends of the target sequence to form a complete sgRNA plasmid through recombination.

The sgRNA fragment is amplified without a template, only requiring a PCR annealing process. The system and program are as follows: PCR reaction system: sgRNA-2F 10 µL, sgRNA-2R 10 µL; PCR reaction program: denaturation at 95 °C for 5 min, annealing at 50 °C for 1 min. After annealing, the target DNA fragment of sgRNA is recovered using a DNA purification kit, and its DNA concentration is measured and diluted to 100 ng/µL.

The pGRB plasmid is digested with Spe I and then dephosphorylated. The sgRNA and the dephosphorylated linearized plasmid are recombined using the Gibson Assembly kit (New England company). Recombination system comprises NEB assembly enzyme 2.5 µL, dephosphorylated linearized plasmid 2 µL, and target DNA fragment of sgRNA 0.5 µL. After assembly at 50 °C for 30 min, the product was transformed into DH5α competent cells. The plasmid is extracted and identified by sequencing using the sequencing primers sgRNA-PF/sgRNA-PR. The plasmid with correct sequencing is named pGRB-sgRNA-2.

The primers used in this experiment are synthesized by Invitrogen (Shanghai). The nucleotides with underlines are the homologous arm sequences of the pGRB cloning vector, and the nucleotides in bold are the sgRNA sequences:

sgRNA-2F:5'-TGACAGCTAGCTCAGTCCTAGGTATAATACTAGTCTATTGATATTATCAA
TACAGTTTTAGAGCTAGAAATAGCAAGTTAAAATAAGG-3' (SEQ ID No.28),

sgRNA-2R:5'-CCTTATTTTAACTTGCTATTTCTAGCTCTAAAACTGTATTGATAATATCA
ATAGACTAGTATTATACCTAGGACTGAGCTAGCTGTCA-3' (SEQ ID No.29).

### II. PCR amplification of homologous recombination fragments

Based on the genome sequence of Escherichia coli W3110 published by NCBI, primers for knocking out the yjiV gene and primers for inserting the Ptrc-ilvE gene and the PilvD-ilvD gene are designed and synthesized. The yjiV gene in the L-valine-producing strain CGMCC22721 and the wild-type Escherichia coli W3110 is knocked out using CRISPR/Cas9 gene editing, and the ilvE gene under the control of the Ptrc promoter (from Bacillus subtilis subsp. subtilis str. 168) and the ilvD gene under the control of the PilvD promoter (from Escherichia coli W3110) are inserted simultaneously.

The primers are as follows (synthesized by Invitrogen, Shanghai):
P9: 5'-TGAATGGACTGCTATGCG-3' (SEQ ID No.30), 
P10: 5'-CACAGTGTATTAAGCAGACGTTAACAACGCAGTACTTCCTGCTG-3' (SEQ ID
No.31),
P11: 5'-GAAGTACTGCGTTGTTAACGTCTGCTTAATACACTGTG-3' (SEQ ID No.32), 
P12:5'-GTATAATGTGTGGAATTGTGAGCGGATAACAATTTCACACAGGAAACAGACC
ATGGAACTTTTTAAATATATGGAG-3' (SEQ ID No.33),
 
P13:5'-CTCACAATTCCACACATTATACGAGCCGGATGATTAATTGTCAATTAACCCCC
CAGTTTCGATTTAT-3' (SEQ ID No.34),
P14: 5'-CCACCAGCACATCCGTTGAAATACAAAAAATGGGAC-3' (SEQ ID No.35),
P15: 5'-TCCCATTTTTTGTATTTCAACGGATGTGCTGGTGG-3' (SEQ ID No.36),
P16: 5'-TGAAGACACGCTGGCTAAC-3' (SEQ ID No.37).

Using the genome DNA of Escherichia coli W3110 as a template, PCR amplification is carried out with primers P9/P10, P13/P14, P15/P16, and KAPA HiFi HotStart to obtain an upper homologous arm of 831 bp, a PilvD-ilvD gene of 1979 bp, and a lower homologous arm fragment of 856 bp. Using the genome DNA of Bacillus subtilis subsp. subtilis str. 168 as a template, PCR amplification is carried out with primers P11/P12 and KAPA HiFi HotStart to obtain a Ptrc-ilvE gene of 1161 bp. After the PCR reaction, agarose gel electrophoresis recovery is carried out using a column-based DNA gel recovery kit. The recovered DNA is subjected to overlap PCR with primers P9/P16 to obtain a homologous recombination DNA fragment △yjiV-Ptrc-ilvE-PilvD-ilvD (SEQ ID No.4) with a size of 4732 bp.

In SEQ ID No.4, positions 3828-3893 represent the PilvD promoter, positions 1977-3827 represent the ilvD gene encoding the ilvD protein set forth in SEQ ID No.5; positions 1903-1976 represent the Ptrc promoter, and positions 808-1902 represent the ilvE gene encoding the ilvE protein set forth in SEQ ID No.6.

### III. Preparation and transformation of competent cells

Competent cells are prepared from CGMCC22721-Cas9 and W3110-Cas9 in Example 1. When the bacterial cells grow to an OD₆₀₀ of 0.1, IPTG with a final concentration of 0.1 mM is added to induce λ-Red-mediated homologous recombination. When the OD₆₀₀ reached 0.4, the bacterial cells are collected to prepare competent cells, and the pGRB-sgRNA-2 plasmid and the homologous recombination DNA fragment △yjiV-Ptrc-ilvE-PilvD-ilvD are transformed respectively. The transformed cells are spread onto 2-YT agar plates containing spectinomycin (100 mg/L) and ampicillin (100 mg/L) and cultured at 32 °C for 12 h. After subculturing the single colonies obtained from the culture, PCR identification is carried out using primers P17/P18. Positive transformants are those with a PCR-amplified fragment of 1543 bp.

The primers are as follows (synthesized by Invitrogen, Shanghai):
P17: 5'-AGCGAGTTTCCAATACCG-3' (SEQ ID No.38),
P18: 5'-ATTTCTCCTGCTTTCGGC-3' (SEQ ID No.39).

The positive transformants are inoculated into 2-YT medium containing spectinomycin (100 mg/L) and arabinose with a final concentration of 0.2% to eliminate the plasmid pGRB-sgRNA-2. Colonies that grow on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT are selected. PCR identification is carried out again using primers P9/P16. Positive strains with an amplified fragment of 4732 bp (SEQ ID No.4) are sent for sequencing. The strains with correct sequencing results are named CGMCC22721-yjiV and W3110-yjiV respectively.

The recombinant strains CGMCC22721-yjiV and W3110-yjiV both lack the yjiV gene and overexpress the ilvE gene under the control of the Ptrc promoter (from Bacillus subtilis subsp. subtilis str. 168) and the ilvD gene under the control of the PilvD promoter (from Escherichia coli W3110). Specifically, in the recombinant strains CGMCC22721-yjiV and W3110-yjiV, the coding region of the yjiV gene on the genomes of the L-valine-producing strain CGMCC22721 and the wild-type Escherichia coli W3110 is knocked out, and the ilvE gene under the control of the Ptrc promoter (from Bacillus subtilis subsp. subtilis str. 168) and the ilvD gene under the control of the PilvD promoter (from Escherichia coli W3110) are inserted, while other nucleotides in their genomes remain unchanged.

### Example 3: Construction of an engineering bacterium with the trpR gene deleted and the ilvH gene overexpressed in the genome

Based on the genome sequence of Escherichia coli W3110 published by NCBI, the CRISPR/Cas9 gene editing technology is used to knock out the trpR gene (confirmed by sequencing that these strains retained intact trpR genes (DNA-binding transcriptional repressor; GeneID: 948917, updated on April 14, 2023) on their chromosomes) in the L-valine-producing strain CGMCC22721 and the wild-type Escherichia coli W3110). Meanwhile, the ilvH^{G14D , S17F} genes (acetolactate synthase) under the control of the Ptrc promoter are inserted to further investigate the effects of these genes on L-valine synthesis.

### I. Construction of sgRNA

Based on the genome sequence of Escherichia coli W3110 published by NCBI, the sgRNA target sequence is designed using CRISPR RGEN Tools (http://www.rgenome.net/cas-designer/). After selecting an appropriate sgRNA target sequence, the homologous arm sequences of the linearized pGRB cloning vector are added at the 5' and 3' ends of the target sequence to form a complete sgRNA plasmid through recombination.

The sgRNA fragment is amplified without a template, only requiring a PCR annealing process. The system and program are as follows: PCR reaction system comprising sgRNA-3F 10 µL and sgRNA-3R 10 µL; and PCR reaction program comprising: denaturation at 95 °C for 5 min, annealing at 50 °C for 1 min. After annealing, the target DNA fragment of sgRNA is recovered using a DNA purification kit, its DNA concentration is measured, and the concentration is diluted to 100 ng/µL.

The pGRB plasmid is digested with SpeI and then dephosphorylated. The sgRNA and the dephosphorylated linearized plasmid are recombined using the Gibson Assembly kit (New England company). Recombination system comprises NEB assembly enzyme 2.5 µL, dephosphorylated linearized plasmid 2 µL, and target DNA fragment of sgRNA 0.5 µL. After assembly at 50 °C for 30 min, the product is transformed into DH5α competent cells. The plasmid is extracted and identified by sequencing using the sequencing primers sgRNA-PF/sgRNA-PR. The plasmid with correct sequencing is named pGRB-sgRNA-3.

The primers used in this experiment (synthesized by Invitrogen, Shanghai) are as follows. The nucleotides with underlines are the homologous arm sequences of the pGRB cloning vector, and the nucleotides in bold are the sgRNA sequences:

sgRNA-3F:5'-TGACAGCTAGCTCAGTCCTAGGTATAATACTAGTCGTGAGTTAAAAAA
TGAA CTGTTTTAGAGCTAGAAATAGCAAGTTAAAATAAGG-3' (SEQ ID No.40),

sgRNA-3R:5'-CCTTATTTTAACTTGCTATTTCTAGCTCTAAAACAGTTCATTTTTTAACT
CA CGACTAGTATTATACCTAGGACTGAGCTAGCTGTCA-3' (SEQ ID No.41).

### II. PCR amplification of homologous recombination fragments

Based on the genome sequence of Escherichia coli W3110 published by NCBI, primers for knocking out the trpR gene and primers for inserting the Ptrc-ilvH^{G14D , S17F} gene sequences are designed and synthesized. The CRISPR/Cas9 gene editing method is used to knock out the trpR gene in the L-valine-producing strain CGMCC22721 and the wild-type Escherichia coli W3110, and simultaneously insert the ilvH^{G14D , S17F} genes under the control of the Ptrc promoter.

The primers (synthesized by Invitrogen, Shanghai) are as follows:
P19: 5'-CCAATCTGGTGAAGAGCAAG-3' (SEQ ID No.42),

P20:5'-CTGTTTCCTGTGTGAAATTGTTATCCGCTCACAATTCCACACATTATACGAGC
CGGA TGATTAATTGTCAATGCTGAATAGGGTGATTGTTG-3' (SEQ ID No.43),

P21:5'-CAATTTCACACAGGAAACAGACCATGCGCCGGATATTATCAGTCTTACTCGA
AAATG AATCAGACGCGTTATTCCGC-3' (SEQ ID No.44),

P22: 5'-GTCTTATCATGCCTACCAAATCAACGCATTATTTTATCG-3' (SEQ ID No.45),
P23: 5'-CGATAAAATAATGCGTTGATTTGGTAGGCATGATAAGAC-3' (SEQ ID No.56),
P24: 5'-GTGCGTCCTAAATCGCTAC-3' (SEQ ID No.47).

Using the genomic DNA of Escherichia coli W3110 as a template, PCR amplification is carried out with primers P19/P20, P21/P22, P23/P24, and KAPA HiFi HotStart to obtain upper homologous arm fragments of 831 bp, Ptrc-ilvH^{G14D , S17F} gene fragments of 535 bp, and lower homologous arm fragments of 801 bp. After the PCR reaction, agarose gel electrophoresis recovery is performed using a column-based DNA gel recovery kit. The recovered DNA is subjected to overlap PCR with primers P19/P24 to obtain the homologous recombination DNA fragment △trpR-Ptrc-ilvH (SEQ ID No.7) with a size of 2108 bp.

In SEQ ID No.7, positions 761-834 represent the Ptrc promoter, and positions 835-1326 represent the ilvH^{G14D , S17F}genes, which encode the ilvH^{G14D , S17F} proteins set forth in in SEQ ID No.8.

### III. Preparation and transformation of competent cells

Competent cells are prepared from CGMCC22721-Cas9 and W3110-Cas9 in Example 1. When the bacteria grow to an OD₆₀₀ of 0.1, IPTG with a final concentration of 0.1 mM is added to induce λ-Red-mediated homologous recombination. When the OD₆₀₀ reached 0.4, the bacteria are collected to prepare competent cells, which are then transformed with the pGRB-sgRNA-3 plasmid and the homologous recombination DNA fragment △trpR-Ptrc-ilvH, respectively. The transformed cells are spread on 2-YT agar plates containing spectinomycin (100 mg/L) and ampicillin (100 mg/L) and cultured at 32 °C for 12 h. After subculturing the single colonies produced by culture, PCR identification is carried out using primers P25/P26. Positive transformants are those with a 1437 bp fragment amplified by PCR.

The primers (synthesized by Invitrogen, Shanghai) are as follows:
P25: 5'-CATCGGCGAAGAGTATGAG-3' (SEQ ID No.48),
P26: 5'-AAAGTCCGACCACACCAGAG-3' (SEQ ID No.49).

The positive transformants are inoculated into 2-YT medium containing spectinomycin (100 mg/L) and arabinose with a final concentration of 0.2% to eliminate the plasmid pGRB-sgRNA-3. Colonies that grow on spectinomycin (100 mg/L) but do not grow on ampicillin (100 mg/L) are selected. These colonies are then transferred to 2-YT medium and cultured at 42 °C to eliminate the pREDCas9 plasmid. Colonies that do not grow on spectinomycin (100 mg/L) but grow on antibiotic-free 2-YT are selected. PCR identification is carried out again using primers P19/P24. Positive strains are those with a 2108 bp (SEQ ID No.7) fragment amplified by PCR. The positive strains are sent for sequencing, and the strains with correct sequencing results are named CGMCC22721-trpR and W3110-trpR, respectively.

The recombinant strains CGMCC22721-trpR and W3110-trpR both lack the trpR gene and overexpress the ilvH^{G14D , S17F} genes under the control of the Ptrc promoter. Specifically, the recombinant strains CGMCC22721-trpR and W3110-trpR are obtained by knocking out part of the coding region of the trpR gene in the genomes of the L-valine-producing strain CGMCC22721 and the wild-type Escherichia coli W3110, respectively, and inserting the mutated ilvH^{G14D , S17F} genes under the control of the Ptrc promoter while keeping other nucleotides in their genomes unchanged.

### Example 4: Construction of an engineering bacterium with a deletion of the lacI-lacZ genes on the genome and overexpression of the DNA polymerase gene

Based on the genome sequence of Escherichia coli W3110 published by NCBI, the CRISPR/Cas9 gene editing technology is used to knock out the lacI-lacZ genes in the L-valine-producing strain CGMCC22721 and the wild-type Escherichia coli W3110 (It is confirmed by sequencing that these strains retain intact lacI-lacZ genes on their chromosomes (DNA-binding transcriptional repressor; beta-D-galactosidase; lacI GeneID: 945007, updated on 2023-04-14; lacZ GeneID: 945006, updated on 2023-04-14)). At the same time, the DNA polymerase gene (from Escherichia coli BL21) initiated by the PxylF promoter is inserted to further investigate the impact of these genes on L-valine synthesis.

### 1. Construction of sgRNA

Based on the genome sequence of Escherichia coli W3110 published by NCBI, the sgRNA target sequence is designed using CRISPR RGEN Tools (http://www.rgenome.net/cas-designer/). After selecting an appropriate sgRNA target sequence, the homologous arm sequences of the linearized pGRB cloning vector are added at the 5' and 3' ends of the target sequence to form a complete sgRNA plasmid through recombination.

The sgRNA fragment is amplified without a template, only requiring a PCR annealing process. The system and program are as follows: PCR reaction system: sgRNA-4F 10 µL, sgRNA-4R 10 µL; PCR reaction program: denaturation at 95 °C for 5 min, annealing at 50 °C for 1 min. After annealing, the target DNA fragment of sgRNA is recovered using a DNA purification kit, its DNA concentration is measured, and the concentration is diluted to 100 ng/µL.

The pGRB plasmid is digested with SpeI and then dephosphorylated. The Gibson Assembly kit (New England Biolabs) is used for the recombination of sgRNA and the dephosphorylated linearized plasmid. Recombination system comprises NEB assembly enzyme 2.5 µL, dephosphorylated linearized plasmid 2 µL, target DNA fragment of sgRNA 0.5 µL. After assembly at 50 °C for 30 min, the product was transformed into DH5α competent cells. The plasmid is extracted and identified by sequencing using the sequencing primers sgRNA-PF/sgRNA-PR. The plasmid with correct sequencing is named pGRB-sgRNA-4.

The primers used in this experiment (synthesized by Invitrogen, Shanghai) are as follows. The underlined nucleotides are the homologous arm sequences of the pGRB cloning vector, and the bold nucleotides are the sgRNA sequences:

sgRNA-4F:5'-TGACAGCTAGCTCAGTCCTAGGTATAATACTAGTTCACTGCCCGCTTTC
CAGTCGTTTTAGAGCTAGAAATAGCAAGTTAAAATAAGG-3' (SEQ ID No.50)

sgRNA-4R:5'-CCTTATTTTAACTTGCTATTTCTAGCTCTAAAACGACTGGAAAGCGGG
CAGTGAACTAGTATTATACCTAGGACTGAGCTAGCTGTCA-3' (SEQ ID No.51)

### 2. PCR amplification of homologous recombination fragments

Based on the genome sequence of Escherichia coli W3110 published by NCBI, primers for knocking out the lacI-lacZ gene sequence and primers for inserting the PxylF-DNA polymerase gene sequence are designed and synthesized. The lacI-lacZ genes in the L-valine-producing strain CGMCC22721 and the wild-type Escherichia coli W3110 are knocked out, and the DNA polymerase gene (from Escherichia coli BL21) initiated by the PxylF promoter is inserted using the CRISPR/Cas9 gene editing method.

The primers (synthesized by Invitrogen, Shanghai) are as follows:
P27: 5'-CGGTAATAATCCACAGCAGG-3' (SEQ ID No.52)
P28: 5'-GACTTCGCGTTCGCGTAACAGGTAGCAGAGCGGGTA-3' (SEQ ID No.53)
P29: 5'-TACCCGCTCTGCTACCTGTTACGCGAACGCGAAGTC-3' (SEQ ID No.54)

P30: 5'-CTAACTACAGAAGGCCCTACACCATGAACACGATTAACATCGC-3' (SEQ ID
No.55)

P31: 5'-GCGATGTTAATCGTGTTCATGGTGTAGGGCCTTCTGTAGTTAG-3' (SEQ ID
No.56)

P32: 5'-CATTAAGTTCTGTCTCGGCGGAGATAATTCACAAGTGTGCG-3' (SEQ ID
No.57)

P33: 5'-CGCACACTTGTGAATTATCTCCGCCGAGACAGAACTTAATG-3' (SEQ ID
No.58)

P34: 5'-GGTTACGGACAGAACTACCG-3' (SEQ ID No.59)

Using the genome DNA of Escherichia coli W3110 as a template, PCR amplification is carried out with primers P27/P28, P31/P32, P33/P34, and KAPA HiFi HotStart to obtain an upper homologous arm of 718 bp, a PxylF promoter of 305 bp, and a lower homologous arm fragment of 928 bp. Using the genome DNA of Escherichia coli BL21 as a template, PCR amplification is carried out with primers P29/P30 and KAPA HiFi HotStart to obtain a DNA polymerase gene of 2693 bp. After the PCR reaction, agarose gel electrophoresis recovery is performed using a column-based DNA gel recovery kit. The recovered DNA is subjected to overlap PCR with primers P27/P34 to obtain the homologous recombination DNA fragment △lacIZ-PxylF-DNA polymerase (SEQ ID No.9) with a size of 4524 bp.

In SEQ ID No.9, the PxylF promoter is shown from positions 3353-3617, and the DNA polymerase gene is shown from positions 701-3352, encoding the DNA polymerase shown in SEQ ID No.10.

### 3. Preparation and transformation of competent cells

Competent cells are prepared from CGMCC22721-Cas9 and W3110-Cas9 in Example 1. When the bacteria grow to an OD₆₀₀ of 0.1, IPTG with a final concentration of 0.1 mM is added to induce λ-Red-mediated homologous recombination. When the OD₆₀₀ reached 0.4, the bacteria are collected to prepare competent cells, and the pGRB-sgRNA-4 plasmid and the homologous recombination DNA fragment △lacIZ-PxylF-DNA polymerase are transformed respectively. The transformed cells are spread on 2-YT agar plates containing spectinomycin (100 mg/L) and ampicillin (100 mg/L) and cultured at 32 °C for 12 h. After subculturing the single colonies obtained from the culture, PCR identification is carried out using primers P35/P36. Positive transformants are those with a 1655 bp fragment amplified by PCR.

The primers (synthesized by Invitrogen, Shanghai) are as follows:
P35: 5'-TTCGCCCATTGTCGTTAC-3' (SEQ ID No.60)
P36: 5'-AGTTCCGCTTACAGCCTACC-3' (SEQ ID No.61)

The positive transformants are inoculated into 2-YT medium containing spectinomycin (100 mg/L) and arabinose with a final concentration of 0.2% to eliminate the plasmid pGRB-sgRNA-4. Colonies that grow on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grow on antibiotic-free 2-YT are selected. PCR identification is carried out again using primers P27/P34. Positive strains with a 4524 bp (SEQ ID No.9) fragment amplified by PCR are sent for sequencing. The strains with correct sequencing results are named CGMCC22721-lacIZ and W3110-lacIZ respectively.

The recombinant strains CGMCC22721-lacIZ and W3110-lacIZ both lack the lacI-lacZ genes and overexpress the DNA polymerase gene (from Escherichia coli BL21) initiated by the PxylF promoter. Specifically, the recombinant strains CGMCC22721-lacIZ and W3110-lacIZ are obtained by knocking out part of the coding region of the lacI-lacZ genes on the genomes of the L-valine-producing strain CGMCC22721 and the wild-type Escherichia coli W3110 respectively, and inserting the DNA polymerase gene (from Escherichia coli BL21) initiated by the PxylF promoter while keeping other nucleotides in their genomes unchanged.

### Example 5: Construction of an engineering bacterium with a deletion of the ycgH gene and overexpression of the ilvC gene in the genome.

Based on the genome sequence of Escherichia coli W3110 published by NCBI, the CRISPR/Cas9 gene editing technology is used to knock out the ycgH gene in the L-valine-producing strain CGMCC22721 and the wild-type Escherichia coli W3110 (it is confirmed by sequencing that these strains retained intact ycgH genes (GeneID: 2847703, updated on April 14, 2023) on their chromosomes). Meanwhile, the ilvC gene (ketol-acid reductoisomerase, from Escherichia coli W3110) driven by the Ptrc promoter is inserted to further investigate the effects of these genes on L-valine synthesis.

### I. Construction of sgRNA

Based on the genome sequence of Escherichia coli W3110 published by NCBI, the sgRNA target sequence is designed using CRISPR RGEN Tools (http://www.rgenome.net/cas-designer/). After selecting an appropriate sgRNA target sequence, the homologous arm sequences of the linearized pGRB cloning vector are added at the 5' and 3' ends of the target sequence to form a complete sgRNA plasmid through recombination.

The sgRNA fragment is amplified without a template, only requiring the PCR annealing process. The system and program are as follows: PCR reaction system: sgRNA-5F 10 µL, sgRNA-5R 10 µL; PCR reaction program: denaturation at 95 °C for 5 min, annealing at 50 °C for 1 min. After annealing, the target DNA fragment of sgRNA is recovered using a DNA purification kit, its DNA concentration is measured, and the concentration is diluted to 100 ng/µL.

The pGRB plasmid is digested with Spe I and then dephosphorylated. The sgRNA and the dephosphorylated linearized plasmid are recombined using the Gibson Assembly kit (New England company). Recombination system comprises: NEB assembly enzyme 2.5 µL, dephosphorylated linearized plasmid 2 µL, and target DNA fragment of sgRNA 0.5 µL. After assembly at 50 °C for 30 min, the product is transformed into DH5α competent cells. The plasmid is extracted and identified by sequencing using the sequencing primers sgRNA-PF/sgRNA-PR. The plasmid with correct sequencing is named pGRB-sgRNA-5.

The primers used in this experiment are as follows (synthesized by Invitrogen, Shanghai). The nucleotides with underlines are the homologous arm sequences of the pGRB cloning vector, and the bold nucleotides are the sgRNA sequences:

sgRNA-5F:5'-TGACAGCTAGCTCAGTCCTAGGTATAATACTAGTCCCGTCCTGCGCCCC
GAA GCGTTTTAGAGCTAGAAATAGCAAGTTAAAATAAGG-3' (SEQ ID No.62),

sgRNA-5R:5'-CCTTATTTTAACTTGCTATTTCTAGCTCTAAAACGCTTCGGGGCGCAGG
ACG GGACTAGTATTATACCTAGGACTGAGCTAGCTGTCA-3' (SEQ ID No.63).

### II. PCR amplification of homologous recombination fragments

Based on the genome sequence of Escherichia coli W3110 published by NCBI, primers for knocking out the ycgH gene and primers for inserting the Ptrc-ilvC gene are designed and synthesized. The ycgH gene in the L-valine-producing strain CGMCC22721 and the wild-type Escherichia coli W3110 is knocked out, and the ilvC gene (from Escherichia coli W3110) driven by the Ptrc promoter is inserted using the CRISPR/Cas9 gene editing method.

The primers are as follows (synthesized by Invitrogen, Shanghai):
P37: 5'-AAATGGAGGGATAACAGCC-3' (SEQ ID No.64),

P38: 5'-GCTGTTGCGGGTTAACGCTCCATTTAGAATAGAGTCGGACGAAAT-3' (SEQ ID
No.65),

P39: 5'-CTAAATGGAGCGTTAACCCGCAACAGCAATAC-3' (SEQ ID No.66),

P40:5'-GCCATTTACGCCAAGTTTTTGACAATTAATCATCCGGCTCGTATAATGTGTGG
AATTGTGAG-3' (SEQ ID No.67),

P41:5'-CAGTGTATTGAAGTAGTTAGCCATGGTCTGTTTCCTGTGTGAAATTGTTATCC
GCTC ACAATTCCACACATTATAC-3' (SEQ ID No.68),

P42: 5'-TTGCCTTCGGGCTTATCTC-3' (SEQ ID No.69).

Using the genomic DNA of Escherichia coli W3110 as a template, PCR amplification is carried out with primers P37/P38, P39/P40, P41/P42, and KAPA HiFi HotStart to obtain an upper homologous arm of 808 bp, a Ptrc-ilvC gene of 1580 bp, and a lower homologous arm fragment of 944 bp. After the PCR reaction, agarose gel electrophoresis recovery is performed using a column-based DNA gel recovery kit. The recovered DNA is subjected to overlap PCR with primers P37/P42 to obtain the homologous recombination DNA fragment △ycgH-Ptrc-ilvC (SEQ ID No.11) with a size of 3189 bp.

In SEQ ID No.11, the Ptrc promoter is shown at positions 2270-2343, and the ilvC gene is shown at positions 794-2269, encoding the ilvC protein shown in SEQ ID No.12.

### III. Preparation of competent cells and transformation

Competent cells are prepared from CGMCC22721-Cas9 and W3110-Cas9 in Example 1. When the bacteria grew to an OD₆₀₀ of 0.1, IPTG with a final concentration of 0.1 mM is added to induce λ-Red-mediated homologous recombination. When the OD₆₀₀ reached 0.4, the bacteria are collected to prepare competent cells, which are then transformed with the pGRB-sgRNA-5 plasmid and the homologous recombination DNA fragment △ycgH-Ptrc-ilvC, respectively, and spread onto 2-YT agar plates containing spectinomycin (100 mg/L) and ampicillin (100 mg/L) and cultured at 32 °C for 12 h. After subculturing the single colonies produced by culture, PCR identification is carried out using primers P43/P44. Positive transformants are those with a 1669 bp fragment amplified by PCR.

The primers are as follows (synthesized by Invitrogen, Shanghai):
P43: 5'-AAGACCTCTGGAAGCGTATC-3' (SEQ ID No.70),
P44: 5'-CGTTGCGGAAGTGAAATC-3' (SEQ ID No.71).

The positive transformants are inoculated into 2-YT medium containing spectinomycin (100 mg/L) and arabinose with a final concentration of 0.2% to eliminate the plasmid pGRB-sgRNA-5. Colonies that grew on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT are selected. PCR identification is carried out again using primers P37/P42. Positive clones with a 3189 bp (SEQ ID No.11) fragment amplified by PCR are sent for sequencing. The strains with correct sequencing results are named CGMCC22721-ycgH and W3110-ycgH, respectively.

The recombinant strains CGMCC22721-ycgH and W3110-ycgH both have a deletion of the ycgH gene and overexpression of the ilvC gene driven by the Ptrc promoter (from Escherichia coli W3110). Specifically, in the recombinant strains CGMCC22721-ycgH and W3110-ycgH, part of the coding region of the ycgH gene on the genomes of the L-valine-producing strain CGMCC22721 and the wild-type Escherichia coli W3110 is knocked out, and the ilvC gene driven by the Ptrc promoter (from Escherichia coli W3110) is inserted, while the other nucleotides in their genomes remain unchanged.

### Example 6: Construction of engineering bacteriums with the yjiV gene deleted and the ilvE-ilvD genes overexpressed in CGMCC22721-yjiT and W3110-yjiT.

Based on the genome sequence of Escherichia coli W3110 published by NCBI, the CRISPR/Cas9 gene editing technology is used to knock out the yjiV gene in the L-valine-producing strain CGMCC22721-yjiT and the wild-type Escherichia coli W3110-yjiT (it is confirmed by sequencing that these strains retained intact yjiV genes on their chromosomes). Meanwhile, the ilvE gene (from Bacillus subtilis subsp. subtilis str. 168) driven by the Ptrc promoter and the ilvD gene (from Escherichia coli W3110) driven by the PilvD promoter are inserted to further investigate the impact of these genes on L-valine synthesis.

### I. PCR Amplification of Homologous Recombination Fragments

Based on the genome sequence of Escherichia coli W3110 published by NCBI, primers for knocking out the yjiV gene and inserting the Ptrc-ilvE and PilvD-ilvD genes are designed and synthesized. The yjiV gene in the genomes of the L-valine-producing strain CGMCC22721-yjiT and the wild-type Escherichia coli W3110-yjiT is knocked out, and the ilvE gene driven by the Ptrc promoter (from Bacillus subtilis) and the ilvD gene driven by the PilvD promoter are inserted using the CRISPR/Cas9 gene editing method.

The primers are as follows (synthesized by Invitrogen, Shanghai):
P45: 5'-AGTTCGCCCTTTGCTCTCTC-3' (SEQ ID No. 72).

Using the genomic DNA of Escherichia coli W3110-yjiT as a template, PCR amplification is performed with primers P45/P10, P13/P14, P15/P16, and KAPA HiFi HotStart to obtain an upper homologous arm of 812 bp, a PilvD-ilvD gene of 1979 bp, and a lower homologous arm of 856 bp. Using the genomic DNA of Bacillus subtilis subsp. subtilis str. 168 as a template, PCR amplification is performed with primers P11/P12 and KAPA HiFi HotStart to obtain a Ptrc-ilvE gene of 1161 bp. After the PCR reactions are completed, agarose gel electrophoresis recovery is performed using a column-based DNA gel recovery kit. The recovered DNA is subjected to overlap PCR with primers P45/P16 to obtain a homologous recombination DNA fragment △yjiV-Ptrc-ilvE-PilvD-ilvD-2 (SEQ ID No. 13) with a size of 4713 bp.

In SEQ ID No. 13, positions 3809-3874 represent the PilvD promoter, positions 1958-3808 represent the ilvD gene encoding the ilvD protein shown in SEQ ID No. 5, positions 1884-1957 represent the Ptrc promoter, and positions 789-1883 represent the ilvE gene encoding the ilvE protein shown in SEQ ID No. 6.

### II. Preparation of Competent Cells and Transformation

The pREDCas9 plasmid (containing a spectinomycin resistance gene) is extracted and transformed into the competent cells of CGMCC22721-yjiT and W3110-yjiT from Example 1. The transformed cells are spread on 2-YT agar plates containing spectinomycin (100 mg/L) and cultured at 32° C. Single colonies resistant to spectinomycin (100 mg/L) are selected and identified by PCR using primers pRedCas9-PF/pRedCas9-PR. Transformants containing the pREDCas9 plasmid, namely CGMCC22721-yjiT-Cas9 and W3110-yjiT-Cas9, are obtained with a PCR product size of 943 bp.

Competent cells of CGMCC22721-yjiT-Cas9 and W3110-yjiT-Cas9 are prepared. When the cell density reached an OD₆₀₀ of 0.1, IPTG is added to a final concentration of 0.1 mM to induce λ-Red-mediated homologous recombination. When the OD₆₀₀ reached 0.4, the cells are harvested to prepare competent cells, which are then transformed with the pGRB-sgRNA-2 plasmid from Example 2 and the homologous recombination DNA fragment △yjiV-Ptrc-ilvE-PilvD-ilvD-2. The transformed cells are spread on 2-YT agar plates containing spectinomycin (100 mg/L) and ampicillin (100 mg/L) and cultured at 32°C for 12 h. Single colonies obtained from the culture are passaged and identified by PCR using primers P46/P18. Positive transformants with a PCR product size of 1561 bp are obtained.

The primers are as follows (synthesized by Invitrogen, Shanghai):
P46: 5'-AGCGTTTCACTCCTACTGGG-3' (SEQ ID No. 73).

The positive transformants are inoculated into 2-YT medium containing spectinomycin (100 mg/L) and arabinose at a final concentration of 0.2% to eliminate the pGRB-sgRNA-2 plasmid. Colonies that grew on spectinomycin (100 mg/L) but not on ampicillin (100 mg/L) are selected. These colonies are then transferred to 2-YT medium and cultured at 42°C to eliminate the pREDCas9 plasmid. Colonies that do not grow on spectinomycin (100 mg/L) but grow on antibiotic-free 2-YT medium are selected. These colonies are further identified by PCR using primers P45/P16. Positive clones with a PCR product size of 4713 bp (SEQ ID No. 13) are sent for sequencing. The strains with correct sequencing results are named CGMCC22721-yjiT-yjiV and W3110-yjiT-yjiV, respectively.

The recombinant strains CGMCC22721-yjiT-yjiV and W3110-yjiT-yjiV both have the yjiT gene deleted and overexpress the brnF-brnE genes (from Corynebacterium glutamicum ATCC13032) driven by the Ptrc promoter; they also have the yjiV gene deleted and overexpress the ilvE gene (from Bacillus subtilis subsp. subtilis str. 168) driven by the Ptrc promoter and the ilvD gene (from Escherichia coli W3110) driven by the PilvD promoter. Specifically, in the recombinant strains CGMCC22721-yjiT-yjiV and W3110-yjiT-yjiV, part of the coding region of the yjiT gene in the genomes of the L-valine-producing strain CGMCC22721 and the wild-type Escherichia coli W3110 is knocked out, and the brnF-brnE genes (from Corynebacterium glutamicum ATCC13032) driven by the Ptrc promoter are inserted. At the same time, part of the coding region of the yjiV gene is knocked out, and the ilvE gene (from Bacillus subtilis subsp. subtilis str. 168) driven by the Ptrc promoter and the ilvD gene (from Escherichia coli W3110) driven by the PilvD promoter are inserted, resulting in recombinant strains with no other nucleotide changes in their genomes.

### Example 7: Construction of engineering bacteriums with the trpR gene deleted and the ilvH gene overexpressed in CGMCC22721-yjiT-yjiV and W3110-yjiT-yjiV.

Based on the genome sequence of Escherichia coli W3110 published by NCBI, the CRISPR/Cas9 gene editing technology is employed to knock out the trpR gene in the L-valine-producing strain CGMCC22721-yjiT-yjiV and the wild-type Escherichia coli W3110-yjiT-yjiV (sequencing confirmed that these strains retained intact trpR genes on their chromosomes). Simultaneously, the ilvH^{G14D , S17F} genes driven by the Ptrc promoter are inserted to further investigate the impact of these genes on L-valine synthesis.

The pREDCas9 plasmid (containing a spectinomycin resistance gene) is extracted and separately transformed into the competent cells of CGMCC22721-yjiT-yjiV and W3110-yjiT-yjiV from Example 6. The transformed cells are spread onto 2-YT agar plates containing spectinomycin (100 mg/L) and incubated at 32°C. Single colonies resistant to spectinomycin (100 mg/L) are selected and subjected to PCR identification using primers pRedCas9-PF/pRedCas9-PR. Transformants with a 943 bp fragment, indicating the presence of the pREDCas9 plasmid, are identified as CGMCC22721-yjiT-yjiV-Cas9 and W3110-yjiT-yjiV-Cas9.

Competent cells of CGMCC22721-yjiT-yjiV-Cas9 and W3110-yjiT-yjiV-Cas9 are prepared. When the cell density reached an OD₆₀₀ of 0.1, IPTG is added to a final concentration of 0.1 mM to induce λ-Red-mediated homologous recombination. When the OD₆₀₀ reached 0.4, the cells are harvested to prepare competent cells, which are then transformed with the pGRB-sgRNA-3 plasmid from Example 3 and the homologous recombination DNA fragment △trpR-Ptrc-ilvH. The transformed cells are spread onto 2-YT agar plates containing spectinomycin (100 mg/L) and ampicillin (100 mg/L) and incubated at 32°C for 12 h. Single colonies obtained from the culture are passaged and identified by PCR using primers P25/P26. Positive transformants with a 1437 bp PCR fragment are identified.

The positive transformants are inoculated into 2-YT medium containing spectinomycin (100 mg/L) and arabinose at a final concentration of 0.2% to eliminate the pGRB-sgRNA-3 plasmid. Colonies that grew on spectinomycin (100 mg/L) but not on ampicillin (100 mg/L) are selected. These colonies are then transferred to 2-YT medium and incubated at 42°C to eliminate the pREDCas9 plasmid. Colonies that do not grow on spectinomycin (100 mg/L) but grew on antibiotic-free 2-YT medium are selected and subjected to PCR identification again using primers P19/P24. Positive clones with a 2108 bp (SEQ ID No.7) PCR fragment are identified and sent for sequencing. The strains with correct sequencing results are named CGMCC22721-yjiT-yjiV-trpR and W3110-yjiT-yjiV-trpR, respectively.

The recombinant strains CGMCC22721-yjiT-yjiV-trpR and W3110-yjiT-yjiV-trpR both lack the yjiT gene and overexpress the brnF-brnE genes driven by the Ptrc promoter (from Corynebacterium glutamicum ATCC13032). They also lack the yjiV gene and overexpress the ilvE gene driven by the Ptrc promoter (from Bacillus subtilis subsp. subtilis str. 168) and the ilvD gene driven by the PilvD promoter (from Escherichia coli W3110). Additionally, they lack the trpR gene and overexpress the ilvH^{G14D , S17F} genes driven by the Ptrc promoter. Specifically, in the recombinant strains CGMCC22721-yjiT-yjiV-trpR and W3110-yjiT-yjiV-trpR, part of the coding region of the yjiT gene in the genomes of the L-valine-producing strain CGMCC22721 and the wild-type Escherichia coli W3110 is knocked out, and the brnF-brnE genes driven by the Ptrc promoter (from Corynebacterium glutamicum ATCC13032) are inserted. Part of the coding region of the yjiV gene is knocked out, and the ilvE gene driven by the Ptrc promoter (from Bacillus subtilis subsp. subtilis str. 168) and the ilvD gene driven by the PilvD promoter (from Escherichia coli W3110) are inserted. Meanwhile, part of the coding region of the trpR gene is knocked out, and the mutant ilvH^{G14D , S17F} genes driven by the Ptrc promoter are inserted, resulting in recombinant strains with all other nucleotides in their genomes remaining unchanged.

### Example 8: Construction of engineering bacteriums with the lacI-lacZ gene deleted and the DNA polymerase gene overexpressed in CGMCC22721-yjiT-yjiV-trpR and W3110-yjiT-yjiV-trpR.

Based on the genome sequence of Escherichia coli W3110 published by NCBI, the CRISPR/Cas9 gene editing technology is utilized to knock out the lacI-lacZ gene in the L-valine-producing strain CGMCC22721-yjiT-yjiV-trpR and the wild-type Escherichia coli W3110-yjiT-yjiV-trpR (sequencing confirmed that these strains retained intact lacI-lacZ genes on their chromosomes). Simultaneously, the DNA polymerase gene driven by the PxylF promoter (from Escherichia coli BL21) is inserted to further investigate the impact of these genes on L-valine synthesis.

The pREDCas9 plasmid (containing a spectinomycin resistance gene) is extracted and separately transformed into the competent cells of CGMCC22721-yjiT-yjiV-trpR and W3110-yjiT-yjiV-trpR from Example 7. The transformed cells are spread onto 2-YT agar plates containing spectinomycin (100 mg/L) and incubated at 32°C. Single colonies resistant to spectinomycin (100 mg/L) are selected and subjected to PCR identification using primers pRedCas9-PF/pRedCas9-PR. Transformants with a 943 bp fragment, indicating the presence of the pREDCas9 plasmid, are identified as CGMCC22721-yjiT-yjiV-trpR-Cas9 and W3110-yjiT-yjiV-trpR-Cas9.

Competent cells of CGMCC22721-yjiT-yjiV trpR-Cas9 and W3110-yjiT-yjiV-trpR-Cas9 are prepared. When the cell density reached an OD₆₀₀ of 0.1, IPTG is added to a final concentration of 0.1 mM to induce λ-Red-mediated homologous recombination. When the OD₆₀₀ reached 0.4, the cells are harvested to prepare competent cells, which are then transformed with the pGRB-sgRNA-4 plasmid from Example 4 and the homologous recombination DNA fragment △lacIZ-PxylF-DNA polymerase. The transformed cells are spread onto 2-YT agar plates containing spectinomycin (100 mg/L) and ampicillin (100 mg/L) and incubated at 32°C for 12 h. Single colonies obtained from the culture are passaged and identified by PCR using primers P35/P36. Positive transformants with a 1655 bp PCR fragment are identified.

The positive transformants are inoculated into 2-YT medium containing spectinomycin (100 mg/L) and arabinose at a final concentration of 0.2% to eliminate the pGRB-sgRNA-4 plasmid. Colonies that grew on spectinomycin (100 mg/L) but not on ampicillin (100 mg/L) are selected. These colonies are then transferred to 2-YT medium and incubated at 42°C to eliminate the pREDCas9 plasmid. Colonies that do not grow on spectinomycin (100 mg/L) but grew on antibiotic-free 2-YT medium are selected and subjected to PCR identification again using primers P27/P34. Positive clones with a 4524 bp (SEQ ID No.9) PCR fragment are identified and sent for sequencing. The strains with correct sequencing results are named CGMCC22721-yjiT-yjiV-trpR-lacIZ and W3110-yjiT-yjiV-trpR-lacIZ, respectively.

The recombinant strains CGMCC22721-yjiT-yjiV-trpR-lacIZ and W3110-yjiT-yjiV-trpR-lacIZ both lack the yjiT gene and overexpressed the brnF-brnE genes driven by the Ptrc promoter (from Corynebacterium glutamicum ATCC13032). They also lack the yjiV gene and overexpressed the ilvE gene driven by the Ptrc promoter (from Bacillus subtilis subsp. subtilis str. 168) and the ilvD gene driven by the PilvD promoter (from Escherichia coli W3110). Additionally, they lack the trpR gene and overexpressed the ilvH^{G14D , S17F} genes driven by the Ptrc promoter. Moreover, they lack the lacI-lacZ gene and overexpressed the DNA polymerase gene driven by the PxylF promoter (from Escherichia coli BL21). Specifically, in the recombinant strains CGMCC22721-yjiT-yjiV-trpR-lacIZ and W3110-yjiT-yjiV-trpR-lacIZ, part of the coding region of the yjiT gene in the genomes of the L-valine-producing strain CGMCC22721 and the wild-type Escherichia coli W3110 is knocked out, and the brnF-brnE genes driven by the Ptrc promoter (from Corynebacterium glutamicum ATCC13032) are inserted. Part of the coding region of the yjiV gene is knocked out, and the ilvE gene driven by the Ptrc promoter (from Bacillus subtilis subsp. subtilis str. 168) and the ilvD gene driven by the PilvD promoter (from Escherichia coli W3110) are inserted. Meanwhile, part of the coding region of the trpR gene is knocked out, and the mutant ilvH^{G14D , S17F} genes driven by the Ptrc promoter are inserted. Furthermore, part of the coding region of the lacI-lacZ gene is knocked out, and the DNA polymerase gene driven by the PxylF promoter (from Escherichia coli BL21) is inserted, resulting in recombinant strains with all other nucleotides in their genomes remaining unchanged.

### Example 9: Construction of engineering bacteriums with the ycgH gene deleted and the ilvC gene overexpressed in CGMCC22721-yjiT-yjiV-trpR-lacIZ and W3110-yjiT-yjiV-trpR-lacIZ.

Based on the genome sequence of Escherichia coli W3110 published by NCBI, the CRISPR/Cas9 gene editing technology is employed to knock out the ycgH gene in the L-valine-producing strain CGMCC22721-yjiT-yjiV-trpR-lacIZ and the wild-type Escherichia coli W3110-yjiT-yjiV-trpR-lacIZ (sequencing confirmed that these strains retained intact ycgH genes on their chromosomes). Simultaneously, the ilvC gene driven by the Ptrc promoter (from Escherichia coli W3110) is inserted to further investigate the impact of these genes on L-valine synthesis.

The pREDCas9 plasmid (containing a spectinomycin resistance gene) is extracted and separately transformed into the competent cells of CGMCC22721-yjiT-yjiV-trpR-lacIZ and W3110-yjiT-yjiV-trpR-lacIZ from Example 8. The transformed cells are spread onto 2-YT agar plates containing spectinomycin (100 mg/L) and incubated at 32°C. Single colonies resistant to spectinomycin (100 mg/L) are selected and subjected to PCR identification using primers pRedCas9-PF/pRedCas9-PR. Transformants with a 943 bp fragment, indicating the presence of the pREDCas9 plasmid, are identified as CGMCC22721-yjiT-yjiV-trpR-lacIZ-Cas9 and W3110-yjiT-yjiV-trpR-lacIZ-Cas9 transformants.

Competent cells of CGMCC22721-yjiT-yjiV-trpR-lacIZ-Cas9 and W3110-yjiT-yjiV-trpR-lacIZ-Cas9 are prepared. When the cell density reached an OD₆₀₀ of 0.1, IPTG is added to a final concentration of 0.1 mM to induce λ-Red-mediated homologous recombination. When the OD₆₀₀ reached 0.4, the cells are harvested to prepare competent cells, which are then transformed with the pGRB-sgRNA-5 plasmid from Example 5 and the homologous recombination DNA fragment △ycgH-Ptrc-ilvC. The transformed cells are spread onto 2-YT agar plates containing spectinomycin (100 mg/L) and ampicillin (100 mg/L) and incubated at 32°C for 12 h. Single colonies obtained from the culture are passaged and identified by PCR using primers P43/P44. Positive transformants with a 1669 bp PCR fragment are identified.

The positive transformants are inoculated into 2-YT medium containing spectinomycin (100 mg/L) and arabinose at a final concentration of 0.2% to eliminate the pGRB-sgRNA-5 plasmid. Colonies that grew on spectinomycin (100 mg/L) but not on ampicillin (100 mg/L) are selected. These colonies are then transferred to 2-YT medium and incubated at 42°C to eliminate the pREDCas9 plasmid. Colonies that do not grow on spectinomycin (100 mg/L) but grew on antibiotic-free 2-YT medium are selected and subjected to PCR identification again using primers P37/P42. Positive clones with a 3189 bp (SEQ ID No.11) PCR fragment are identified and sent for sequencing. The strains with correct sequencing results are named CGMCC22721-yjiT-yjiV-trpR-lacIZ-ycgH and W3110-yjiT-yjiV-trpR-lacIZ-ycgH, respectively.

The recombinant strains CGMCC22721-yjiT-yjiV-trpR-lacIZ-ycgH and W3110-yjiT-yjiV-trpR-lacIZ-ycgH both lack the yjiT gene and overexpress the brnF-brnE genes driven by the Ptrc promoter (from Corynebacterium glutamicum ATCC13032). They also lack the yjiV gene and overexpress the ilvE gene driven by the Ptrc promoter (from Bacillus subtilis subsp. subtilis str. 168) and the ilvD gene driven by the PilvD promoter (from Escherichia coli W3110). Additionally, they lack the trpR gene and overexpress the ilvH^{G14D , S17F} genes driven by the Ptrc promoter. Moreover, they lack the lacI-lacZ gene and overexpress the DNA polymerase gene driven by the PxylF promoter (from Escherichia coli BL21). Furthermore, they lack the ycgH gene and overexpress the ilvC gene driven by the Ptrc promoter (from Escherichia coli W3110). Specifically, in the recombinant strains CGMCC22721-yjiT-yjiV-trpR-lacIZ-ycgH and W3110-yjiT-yjiV-trpR-lacIZ-ycgH, part of the coding region of the yjiT gene in the genomes of the L-valine-producing strain CGMCC22721 and the wild-type Escherichia coli W3110 is knocked out, and the brnF-brnE genes driven by the Ptrc promoter (from Corynebacterium glutamicum ATCC13032) are inserted. Part of the coding region of the yjiV gene is knocked out, and the ilvE gene driven by the Ptrc promoter (from Bacillus subtilis subsp. subtilis str. 168) and the ilvD gene driven by the PilvD promoter (from Escherichia coli W3110) are inserted. Meanwhile, part of the coding region of the trpR gene is knocked out, and the mutant ilvH^{G14D , S17F} genes driven by the Ptrc promoter are inserted. Part of the coding region of the lacI-lacZ gene is knocked out, and the DNA polymerase gene driven by the PxylF promoter (from Escherichia coli BL21) is inserted. Additionally, part of the coding region of the ycgH gene is knocked out, and the ilvC gene driven by the Ptrc promoter (from Escherichia coli W3110) is inserted, resulting in recombinant strains with all other nucleotides in their genomes remaining unchanged.

### Example 10: Construction of engineering bacteriums with the trpR gene deleted and the ilvH gene overexpressed in CGMCC22721-yjiV and W3110-yjiV.

Based on the genome sequence of Escherichia coli W3110 published by NCBI, the CRISPR/Cas9 gene editing technology is used to knock out the trpR gene in the L-valine-producing strain CGMCC22721-yjiV and the wild-type Escherichia coli W3110-yjiV (it is confirmed by sequencing that these strains retained intact trpR genes on their chromosomes). At the same time, the ilvH^{G14D , S17F} genes driven by the Ptrc promoter are inserted to further investigate the effects of these genes on L-valine synthesis.

The pREDCas9 plasmid (containing a spectinomycin resistance gene) is extracted and separately transformed into the competent cells of CGMCC22721-yjiV and W3110-yjiV from Example 2. The transformed cells are spread on 2-YT agar plates containing spectinomycin (100 mg/L) and cultured at 32 °C. Single colonies resistant to spectinomycin (100 mg/L) are selected and subjected to PCR identification using primers pRedCas9-PF/pRedCas9-PR. Transformants containing the pREDCas9 plasmid, namely CGMCC22721-yjiV-Cas9 and W3110-yjiV-Cas9, are obtained, producing a 943 bp fragment.

Competent cells of CGMCC22721-yjiV-Cas9 and W3110-yjiV-Cas9 are prepared. When the cell density reached an OD₆₀₀ of 0.1, IPTG is added to a final concentration of 0.1 mM to induce λ-Red-mediated homologous recombination. When the OD₆₀₀ reached 0.4, the cells are harvested to prepare competent cells, which are then separately transformed with the pGRB-sgRNA-3 plasmid from Example 3 and the homologous recombination DNA fragment △trpR-Ptrc-ilvH. The transformed cells are spread on 2-YT agar plates containing spectinomycin (100 mg/L) and ampicillin (100 mg/L) and cultured at 32 °C for 12 h. After subculturing the single colonies obtained from the culture, PCR identification is performed using primers P25/P26. Positive transformants are identified by the amplification of a 1437 bp fragment.

The positive transformants are inoculated into 2-YT medium containing spectinomycin (100 mg/L) and arabinose at a final concentration of 0.2% to eliminate the pGRB-sgRNA-3 plasmid. Colonies that grew on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT medium are selected. PCR identification is performed again using primers P19/P24, and positive colonies that amplified a 2108 bp fragment (SEQ ID No.7) are identified. The positive colonies are sent for sequencing, and the strains with correct sequencing results are named CGMCC22721-yjiV-trpR and W3110-yjiV-trpR, respectively.

The recombinant strains CGMCC22721-yjiV-trpR and W3110-yjiV-trpR both lack the yjiV gene and overexpress the ilvE gene driven by the Ptrc promoter (from Bacillus subtilis subsp. subtilis str.168) and the ilvD gene driven by the PilvD promoter (from Escherichia coli W3110). They also lack the trpR gene and overexpress the mutated ilvH^{G14D , S17F} genes driven by the Ptrc promoter. Specifically, in the recombinant strains CGMCC22721-yjiV-trpR and W3110-yjiV-trpR, part of the coding region of the yjiV gene on the genomes of the L-valine-producing strain CGMCC22721 and the wild-type Escherichia coli W3110 is knocked out, and the ilvE gene driven by the Ptrc promoter (from Bacillus subtilis subsp. subtilis str.168) and the ilvD gene driven by the PilvD promoter (from Escherichia coli W3110) are inserted. At the same time, part of the coding region of the trpR gene is knocked out, and the mutated ilvH^{G14D , S17F} genes driven by the Ptrc promoter are inserted, while the other nucleotides in their genomes remain unchanged, resulting in the recombinant strains.

### Example 11: Construction of engineering bacteriums with the lacI-lacZ genes deleted and the DNA polymerase gene overexpressed in CGMCC22721-yjiV-trpR and W3110-yjiV-trpR.

Based on the genome sequence of Escherichia coli W3110 published by NCBI, the CRISPR/Cas9 gene editing technology is employed to knock out the lacI-lacZ genes in the L-valine-producing strain CGMCC22721-yjiV-trpR and the wild-type Escherichia coli W3110-yjiV-trpR (it is confirmed by sequencing that these strains retained intact lacI-lacZ genes on their chromosomes). Simultaneously, the DNA polymerase gene driven by the PxylF promoter (from Escherichia coli BL21) is inserted to further investigate the impact of these genes on L-valine synthesis.

The pREDCas9 plasmid (containing a spectinomycin resistance gene) is extracted and separately transformed into the competent cells of CGMCC22721-yjiV-trpR and W3110-yjiV-trpR from Example 10. The transformed cells are spread on 2-YT agar plates containing spectinomycin (100 mg/L) and cultured at 32 ° C. Single colonies resistant to spectinomycin (100 mg/L) are selected and subjected to PCR identification using primers pRedCas9-PF/pRedCas9-PR. Transformants containing the pREDCas9 plasmid, namely CGMCC22721-yjiV-trpR-Cas9 and W3110-yjiV-trpR-Cas9, are obtained, producing a 943 bp fragment.

Competent cells of CGMCC22721-yjiV-trpR-Cas9 and W3110-yjiV-trpR-Cas9 are prepared. When the cell density reached an OD₆₀₀ of 0.1, IPTG is added to a final concentration of 0.1 mM to induce λ -Red-mediated homologous recombination. When the OD₆₀₀ reached 0.4, the cells are harvested to prepare competent cells, which are then separately transformed with the pGRB-sgRNA-4 plasmid from Example 4 and the homologous recombination DNA fragment △lacIZ-PxylF-DNA polymerase. The transformed cells are spread on 2-YT agar plates containing spectinomycin (100 mg/L) and ampicillin (100 mg/L) and cultured at 32 °C for 12 h. After subculturing the single colonies obtained from the culture, PCR identification is performed using primers P35/P36. Positive transformants are identified by the amplification of a 1655 bp fragment.

The positive transformants are inoculated into 2-YT medium containing spectinomycin (100 mg/L) and arabinose at a final concentration of 0.2% to eliminate the pGRB-sgRNA-4 plasmid. Colonies that grew on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT medium are selected. PCR identification is performed again using primers P27/P34, and positive colonies that amplified a 4524 bp fragment (SEQ ID No.9) are identified. The positive colonies are sent for sequencing, and the strains with correct sequencing results are named CGMCC22721-yjiV-trpR-lacIZ and W3110-yjiV-trpR-lacIZ, respectively.

The recombinant strains CGMCC22721-yjiV trpR-lacIZ and W3110-yjiV-trpR-lacIZ both lack the yjiV gene and overexpress the ilvE gene driven by the Ptrc promoter (from Bacillus subtilis subsp. subtilis str.168) and the ilvD gene driven by the PilvD promoter (from Escherichia coli W3110). They also lack the trpR gene and overexpress the mutated ilvH^{G14D , S17F} genes driven by the Ptrc promoter. Additionally, they lack the lacI-lacZ genes and overexpress the DNA polymerase gene driven by the PxylF promoter (from Escherichia coli BL21). Specifically, in the recombinant strains CGMCC22721-yjiV-trpR-lacIZ and W3110-yjiV-trpR-lacIZ, part of the coding region of the yjiV gene on the genomes of the L-valine-producing strain CGMCC22721 and the wild-type Escherichia coli W3110 is knocked out, and the ilvE gene driven by the Ptrc promoter (from Bacillus subtilis subsp. subtilis str.168) and the ilvD gene driven by the PilvD promoter (from Escherichia coli W3110) are inserted. Part of the coding region of the trpR gene is knocked out, and the mutated ilvH^{G14D , S17F} genes driven by the Ptrc promoter are inserted. At the same time, part of the coding region of the lacI-lacZ genes is knocked out, and the DNA polymerase gene driven by the PxylF promoter (from Escherichia coli BL21) is inserted, resulting in the recombinant strains with no changes in the other nucleotides in their genomes.

### Example 12: Construction of engineering bacteriums with the ycgH gene deleted and the ilvC gene overexpressed in CGMCC22721-yjiV-trpR-lacIZ and W3110-yjiV-trpR-lacIZ.

Based on the genome sequence of Escherichia coli W3110 published by NCBI, the CRISPR/Cas9 gene editing technology is employed to knock out the ycgH gene in the L-valine-producing strain CGMCC22721-yjiV-trpR-lacIZ and the wild-type Escherichia coli W3110-yjiV-trpR-lacIZ (it is confirmed by sequencing that these strains retained intact ycgH genes on their chromosomes). Simultaneously, the ilvC gene driven by the Ptrc promoter (from Escherichia coli W3110) is inserted to further investigate the impact of these genes on L-valine synthesis.

The pREDCas9 plasmid (containing a spectinomycin resistance gene) is extracted and separately transformed into the competent cells of CGMCC22721-yjiV-trpR-lacIZ from Example 11 and the wild-type Escherichia coli W3110-yjiV-trpR-lacIZ. The transformed cells are spread on 2-YT agar plates containing spectinomycin (100 mg/L) and cultured at 32 °C. Single colonies resistant to spectinomycin (100 mg/L) are selected and subjected to PCR identification using primers pRedCas9-PF/pRedCas9-PR. Transformants containing the pREDCas9 plasmid, namely CGMCC22721-yjiV-trpR-lacIZ-Cas9 and W3110-yjiV-trpR-lacIZ-Cas9, are obtained, producing a 943 bp fragment.

Competent cells of CGMCC22721-yjiV-trpR-lacIZ-Cas9 and W3110-yjiV-trpR-lacIZ-Cas9 are prepared. When the cell density reached an OD₆₀₀ of 0.1, IPTG is added to a final concentration of 0.1 mM to induce λ -Red-mediated homologous recombination. When the OD₆₀₀ reached 0.4, the cells are harvested to prepare competent cells, which are then separately transformed with the pGRB-sgRNA-5 plasmid from Example 5 and the homologous recombination DNA fragment △ycgH-Ptrc-ilvC. The transformed cells are spread on 2-YT agar plates containing spectinomycin (100 mg/L) and ampicillin (100 mg/L) and cultured at 32 °C for 12 h. After subculturing the single colonies obtained from the culture, PCR identification is performed using primers P43/P44. Positive transformants are identified by the amplification of a 1669 bp fragment.

The positive transformants are inoculated into 2-YT medium containing spectinomycin (100 mg/L) and arabinose at a final concentration of 0.2% to eliminate the pGRB-sgRNA-5 plasmid. Colonies that grew on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT medium are selected. PCR identification is performed again using primers P37/P42, and positive colonies that amplified a 3189 bp fragment (SEQ ID No.11) are identified. The positive colonies are sent for sequencing, and the strains with correct sequencing results are named CGMCC22721-yjiV-trpR-lacIZ-ycgH and W3110-yjiV-trpR-lacIZ-ycgH, respectively.

The recombinant strains CGMCC22721-yjiV-trpR-lacIZ-ycgH and W3110-yjiV-trpR-lacIZ-ycgH both lack the yjiV gene and overexpress the ilvE gene driven by the Ptrc promoter (from Bacillus subtilis subsp. subtilis str.168) and the ilvD gene driven by the PilvD promoter (from Escherichia coli W3110). They also lack the trpR gene and overexpress the mutated ilvH^{G14D , S17F} genes driven by the Ptrc promoter. Additionally, they lack the lacI-lacZ genes and overexpress the DNA polymerase gene driven by the PxylF promoter (from Escherichia coli BL21). Moreover, they lack the ycgH gene and overexpress the ilvC gene driven by the Ptrc promoter (from Escherichia coli W3110). Specifically, in the recombinant strains CGMCC22721-yjiV-trpR-lacIZ-ycgH and W3110-yjiV-trpR-lacIZ-ycgH, part of the coding region of the yjiV gene on the genomes of the L-valine-producing strain CGMCC22721 and the wild-type Escherichia coli W3110 is knocked out, and the ilvE gene driven by the Ptrc promoter (from Bacillus subtilis subsp. subtilis str.168) and the ilvD gene driven by the PilvD promoter (from Escherichia coli W3110) are inserted. Part of the coding region of the trpR gene is knocked out, and the mutated ilvH^{G14D , S17F} genes driven by the Ptrc promoter are inserted. Part of the coding region of the lacI-lacZ genes is knocked out, and the DNA polymerase gene driven by the PxylF promoter (from Escherichia coli BL21) is inserted. At the same time, part of the coding region of the ycgH gene is knocked out, and the ilvC gene driven by the Ptrc promoter (from Escherichia coli W3110) is inserted, resulting in the recombinant strains with no changes in the other nucleotides in their genomes.

### Example 13: Construction of engineering bacteriums with the lacI-lacZ gene deleted and the DNA polymerase gene overexpressed in CGMCC22721-trpR and W3110-trpR.

Based on the genome sequence of Escherichia coli W3110 published by NCBI, the CRISPR/Cas9 gene editing technology is utilized to knock out the lacI-lacZ gene in the L-valine-producing strain CGMCC22721-trpR and the wild-type Escherichia coli W3110-trpR (it is confirmed by sequencing that these strains retained intact lacI-lacZ genes on their chromosomes). Simultaneously, the DNA polymerase gene driven by the PxylF promoter (from Escherichia coli BL21) is inserted to further investigate the impact of these genes on L-valine synthesis.

The pREDCas9 plasmid (containing a spectinomycin resistance gene) is extracted and separately transformed into the competent cells of CGMCC22721-trpR and W3110-trpR from Example 3. The transformed cells are spread on 2-YT agar plates containing spectinomycin (100 mg/L) and cultured at 32 °C. Single colonies resistant to spectinomycin (100 mg/L) are selected and subjected to PCR identification using primers pRedCas9-PF/pRedCas9-PR. Transformants containing the pREDCas9 plasmid, namely CGMCC22721-trpR-Cas9 and W3110-trpR-Cas9, are obtained, producing a 943 bp fragment.

Competent cells of CGMCC22721-trpR-Cas9 and W3110-trpR-Cas9 are prepared. When the cell density reached an OD₆₀₀ of 0.1, IPTG is added to a final concentration of 0.1 mM to induce λ-Red-mediated homologous recombination. When the OD₆₀₀ reached 0.4, the cells are harvested to prepare competent cells, which are then separately transformed with the pGRB-sgRNA-4 plasmid from Example 4 and the homologous recombination DNA fragment △lacIZ-PxylF-DNA polymerase. The transformed cells are spread on 2-YT agar plates containing spectinomycin (100 mg/L) and ampicillin (100 mg/L) and cultured at 32 °C for 12 h. After subculturing the single colonies obtained from the culture, PCR identification is performed using primers P35/P36. Positive transformants are identified by the amplification of a 1655 bp fragment.

The positive transformants are inoculated into 2-YT medium containing spectinomycin (100 mg/L) and arabinose at a final concentration of 0.2% to eliminate the pGRB-sgRNA-4 plasmid. Colonies that grew on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT medium are selected. PCR identification is performed again using primers P27/P34, and positive colonies that amplified a 4524 bp fragment (SEQ ID No.9) are identified. The positive colonies are sent for sequencing, and the strains with correct sequencing results are named CGMCC22721-trpR-lacIZ and W3110-trpR-lacIZ, respectively.

The recombinant strains CGMCC22721-trpR-lacIZ and W3110-trpR-lacIZ both lack the trpR gene and overexpress the mutated ilvH^{G14D , S17F} genes driven by the Ptrc promoter. They also lack the lacI-lacZ genes and overexpress the DNA polymerase gene driven by the PxylF promoter (from Escherichia coli BL21). Specifically, in the recombinant strains CGMCC22721-trpR-lacIZ and W3110-trpR-lacIZ, part of the coding region of the trpR gene on the genomes of the L-valine-producing strain CGMCC22721 and the wild-type Escherichia coli W3110 is knocked out, and the mutated ilvH^{G14D , S17F} genes driven by the Ptrc promoter are inserted. At the same time, part of the coding region of the lacI-lacZ genes is knocked out, and the DNA polymerase gene driven by the PxylF promoter (from Escherichia coli BL21) is inserted, resulting in the recombinant strains with no changes in the other nucleotides in their genomes.

### Example 14: Construction of engineering bacteriums with the ycgH gene deleted and the ilvC gene overexpressed in CGMCC22721-trpR-lacIZ and W3110-trpR-lacIZ.

Based on the genome sequence of Escherichia coli W3110 published by NCBI, the CRISPR/Cas9 gene editing technology is employed to knock out the ycgH gene in the L-valine-producing strain CGMCC22721-trpR-lacIZ and the wild-type Escherichia coli W3110-trpR-lacIZ (it is confirmed by sequencing that these strains retained intact ycgH genes on their chromosomes). Simultaneously, the ilvC gene driven by the Ptrc promoter (from Escherichia coli W3110) is inserted to further investigate the impact of these genes on L-valine synthesis.

The pREDCas9 plasmid (containing a spectinomycin resistance gene) is extracted and separately transformed into the competent cells of CGMCC22721-trpR-lacIZ and the wild-type Escherichia coli W3110-trpR-lacIZ from Example 13. The transformed cells are spread on 2-YT agar plates containing spectinomycin (100 mg/L) and cultured at 32 °C. Single colonies resistant to spectinomycin (100 mg/L) are selected and subjected to PCR identification using primers pRedCas9-PF/pRedCas9-PR. Transformants containing the pREDCas9 plasmid, namely CGMCC22721-trpR-lacIZ-Cas9 and W3110-trpR-lacIZ-Cas9, are obtained, producing a 943 bp fragment.

Competent cells of CGMCC22721-trpR-lacIZ-Cas9 and W3110-trpR-lacIZ-Cas9 are prepared. When the cell density reached an OD₆₀₀ of 0.1, IPTG is added to a final concentration of 0.1 mM to induce λ -Red-mediated homologous recombination. When the OD₆₀₀ reached 0.4, the cells are harvested to prepare competent cells, which are then separately transformed with the pGRB-sgRNA-5 plasmid from Example 5 and the homologous recombination DNA fragment ΔycgH-Ptrc-ilvC. The transformed cells are spread on 2-YT agar plates containing spectinomycin (100 mg/L) and ampicillin (100 mg/L) and cultured at 32 °C for 12 h. After subculturing the single colonies obtained from the culture, PCR identification is performed using primers P43/P44. Positive transformants are identified by the amplification of a 1669 bp fragment.

The positive transformants are inoculated into 2-YT medium containing spectinomycin (100 mg/L) and arabinose at a final concentration of 0.2% to eliminate the pGRB-sgRNA-5 plasmid. Colonies that grew on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT medium are selected. PCR identification is performed again using primers P37/P42, and positive colonies that amplified a 3189 bp fragment (SEQ ID No.11) are identified. The positive colonies are sent for sequencing, and the strains with correct sequencing results are named CGMCC22721-trpR-lacIZ-ycgH and W3110-trpR-lacIZ-ycgH, respectively.

The recombinant strains CGMCC22721-trpR-lacIZ-ycgH and W3110-trpR-lacIZ-ycgH both lack the trpR gene and overexpress the mutated ilvH^{G14D , S17F} genes driven by the Ptrc promoter. They also lack the lacI-lacZ genes and overexpress the DNA polymerase gene driven by the PxylF promoter (from Escherichia coli BL21). Additionally, they lack the ycgH gene and overexpress the ilvC gene driven by the Ptrc promoter (from Escherichia coli W3110). Specifically, in the recombinant strains CGMCC22721-trpR-lacIZ-ycgH and W3110-trpR-lacIZ-ycgH, part of the coding region of the trpR gene on the genomes of the L-valine-producing strain CGMCC22721 and the wild-type Escherichia coli W3110 is knocked out, and the mutated ilvH^{G14D , S17F} genes driven by the Ptrc promoter are inserted. Part of the coding region of the lacI-lacZ genes is knocked out, and the DNA polymerase gene driven by the PxylF promoter (from Escherichia coli BL21) is inserted. At the same time, part of the coding region of the ycgH gene is knocked out, and the ilvC gene driven by the Ptrc promoter (from Escherichia coli W3110) is inserted, resulting in the recombinant strains with no changes in the other nucleotides in their genomes.

### Example 15: Construction of engineering bacteriums with the ycgH gene deleted and the ilvC gene overexpressed in CGMCC22721-lacIZ and W3110-lacIZ.

Based on the genome sequence of Escherichia coli W3110 published by NCBI, the CRISPR/Cas9 gene editing technology is utilized to knock out the ycgH gene in the L-valine-producing strain CGMCC22721-lacIZ and the wild-type Escherichia coli W3110-lacIZ (it is confirmed by sequencing that these strains retained intact ycgH genes on their chromosomes). Simultaneously, the ilvC gene driven by the Ptrc promoter (from Escherichia coli W3110) is inserted to further investigate the impact of these genes on L-valine synthesis.

The pREDCas9 plasmid (containing a spectinomycin resistance gene) is extracted and separately transformed into the competent cells of CGMCC22721-lacIZ from Example 4 and the wild-type Escherichia coli W3110-lacIZ. The transformed cells are spread on 2-YT agar plates containing spectinomycin (100 mg/L) and cultured at 32 °C. Single colonies resistant to spectinomycin (100 mg/L) are selected and subjected to PCR identification using primers pRedCas9-PF/pRedCas9-PR. Transformants containing the pREDCas9 plasmid, namely CGMCC22721-lacIZ-Cas9 and W3110-lacIZ-Cas9, are obtained, producing a 943 bp fragment.

Competent cells of CGMCC22721-lacIZ-Cas9 and W3110-lacIZ-Cas9 are prepared. When the cell density reached an OD₆₀₀ of 0.1, IPTG is added to a final concentration of 0.1 mM to induce λ -Red-mediated homologous recombination. When the OD₆₀₀ reached 0.4, the cells are harvested to prepare competent cells, which are then separately transformed with the pGRB-sgRNA-5 plasmid from Example 5 and the homologous recombination DNA fragment △ycgH-Ptrc-ilvC. The transformed cells are spread on 2-YT agar plates containing spectinomycin (100 mg/L) and ampicillin (100 mg/L) and cultured at 32 °C for 12 h. After subculturing the single colonies obtained from the culture, PCR identification is performed using primers P43/P44. Positive transformants are identified by the amplification of a 1669 bp fragment.

The positive transformants are inoculated into 2-YT medium containing spectinomycin (100 mg/L) and arabinose at a final concentration of 0.2% to eliminate the pGRB-sgRNA-5 plasmid. Colonies that grew on spectinomycin (100 mg/L) but not on ampicillin (100 mg/L) are selected. These colonies are then transferred to 2-YT medium and cultured at 42 °C to eliminate the pREDCas9 plasmid. Colonies that do not grow on spectinomycin (100 mg/L) but grew on antibiotic-free 2-YT medium are selected. PCR identification is performed again using primers P37/P42, and positive colonies that amplified a 3189 bp fragment (SEQ ID No.11) are identified. The positive colonies are sent for sequencing, and the strains with correct sequencing results are named CGMCC22721-lacIZ-ycgH and W3110-lacIZ-ycgH, respectively.

The recombinant strains CGMCC22721-lacIZ-ycgH and W3110-lacIZ-ycgH both lack the lacI-lacZ genes and overexpress the DNA polymerase gene driven by the PxylF promoter (from Escherichia coli BL21). They also lack the ycgH gene and overexpress the ilvC gene driven by the Ptrc promoter (from Escherichia coli W3110). Specifically, in the recombinant strains CGMCC22721-lacIZ-ycgH and W3110-lacIZ-ycgH, part of the coding region of the lacI-lacZ genes on the genomes of the L-valine-producing strain CGMCC22721 and the wild-type Escherichia coli W3110 is knocked out, and the DNA polymerase gene driven by the PxylF promoter (from Escherichia coli BL21) is inserted. At the same time, part of the coding region of the ycgH gene is knocked out, and the ilvC gene driven by the Ptrc promoter (from Escherichia coli W3110) is inserted, resulting in the recombinant strains with no changes in the other nucleotides in their genomes.

### Example 16: L-Valine Fermentation Experiment.

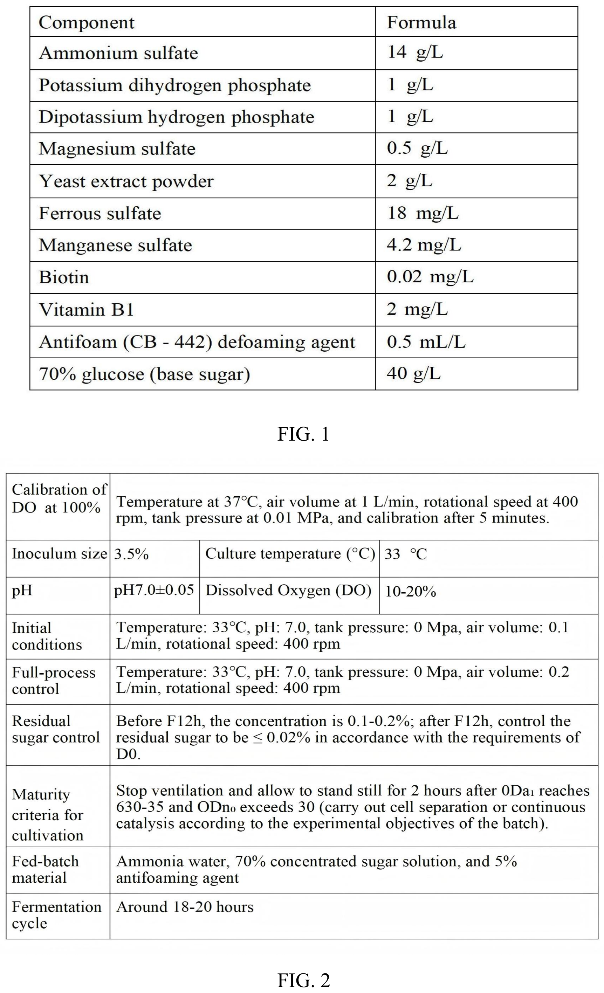

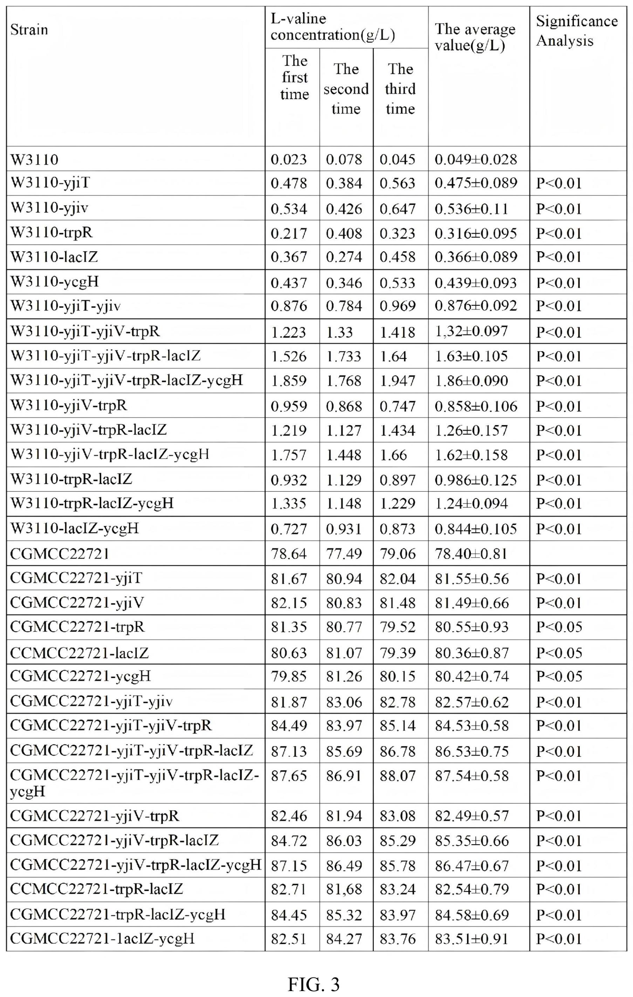

The strains constructed in Examples 1 to 15, along with the L-valine-producing strain CGMCC22721 and the wild-type Escherichia coli W3110, are subjected to fermentation experiments in a fermenter of model BLBIO-5GC-4-H (Shanghai Bailun Biotechnology Co., Ltd.) using the medium shown in FIG. 1 and the control process depicted in FIG. 2. Each strain is tested in triplicate, and the results are presented in FIG. 3. As demonstrated by the above fermentation results, both for the L-valine-producing strain CGMCC22721 and the model strain, the wild-type Escherichia coli W3110, the modifications made in Examples 1 to 15 contributed to an increase in L-valine yield.

The present disclosure has been described in detail above. For those skilled in the art, it is possible to implement the present disclosure within a broad scope under equivalent parameters, concentrations, and conditions without departing from the spirit and scope of the disclosure and without the need for unnecessary experimentation. Although specific embodiments of the present disclosure have been provided, it should be understood that further improvements to the disclosure can be made. In summary, in accordance with the principles of the present disclosure, this application intends to encompass any modifications, uses, or improvements to the disclosure, including changes made using conventional techniques known in the art that deviate from the scope disclosed in this application.

### Industrial Applicability

Experiments have demonstrated that the engineering bacteriums modified according to the present disclosure can enhance L-valine yield and are applicable for the production of L-valine, exhibiting excellent application prospects.

### Free Content of Sequence Listing

SEQ ID No.1: Homologous recombination DNA fragment △yjiT-Ptrc-brnF-brnE sequence (2579bp), artificial sequence 
1 GAGTGATGAG CGGTTGAAGG ATATCGGGTT ACGCAGGGAG GATGTGGAGT GAGGGGGGAT

61 ATAGATTTAT ATATAATAAA ACGTTTTTAT GTTTTTAAAT TAAGTTATAA AAATTTTCCC

121 GAGACAATTC ATCAATAGGT ATGGAGTGTA CAGGAATATC TTCTTCACTA ACCTCTTTAA
 
181 ACTCATGTCG ATAACGGTCA AAAAACGCAC AGTGATTACT TGTGTTTTTT GACTGACTTT

241 ACAACTCTCA TTATTCGCTA TTGTGCAGTT TCTCTAATTG TTTTATACCC TGGAAAGTTA

301 AATGTCAGCT ACTGAATACT TTTTGATTGT TTGAGATTTA TTTTCATTTG AAATTATAAA

361 ATCAGGTGAT AAATGAGTTG TGATTAATAG ATGGTTGATA TCATTTTTAT CTAAAATTGA

421 TTTATAGTAT CGACCTGAAA AAATAGTTGT TGCCGCCTGA GTAACTATAC AATATTCTGA

481 AAGGTTTTCT TTCAAATTAG AAATGTTGTG GGGTTTTGTT TTTGTATCTT TTATCTCTAA

541 GGAGCTTGCT TTGGGTCAAT CAGAATACAT TTCATGGGTA AAATGTACTT CCTGGCTAAG

601 TAACTTTGTG AATCTTAGAG GGTTGAGACA ACCGGATGGC CGTCCTCTTT ATGAATATCA

661 TGCAACCAAT GATGAATATA CCCAATTAAC GCAGCTACTC CGTGCAGTCG GTCAATCACA

721 ATCTAATATA TGTAATAGGG ACTTTGCTGC CTGTTTTTTG ACAATTAATC ATCCGGCTCG

781 TATAATGTGT GGAATTGTGA GCGGATAACA ATTTCACACA GGAAACAGAC CGTGCAAAAA

841 ACGCAAGAGA TTCATTCAAG CCTGGAGGTG TCGCCATCCA AGGCAGCCCT GGAACCAGAT

901 GATAAAGGTT ATCGGCGCTA CGAAATCGCG CAAGGTCTAA AAACCTCCCT TGCTGCAGGT

961 TTGGGCATGT ACCCGATTGG TATTGCGTTT GGTCTCTTGG TTATTCAATA CGGCTACGAA

1021 TGGTGGGCAG CCCCACTGTT TTCCGGCCTG ATTTTCGCGG GCTCCACCGA AATGCTGGTC

1081 ATCGCCCTCG TTGTGGGCGC AGCGCCCCTG GGCGCCATCG CGCTCACCAC ATTGCTGGTG

1141 AACTTCCGCC ACGTATTCTA TGCGTTTTCA TTCCCGCTGC ATGTGGTCAA AAACCCCATT

1201 GCCCGTTTCT ATTCGGTTTT CGCGCTTATC GACGAAGCCT ACGCAGTCAC TGCGGCCAGG

1261 CCCGCAGGCT GGTCGGCGTG GCGACTTATC TCAATGCAAA TAGCGTTTCA CTCCTACTGG

1321 GTATTCGGCG GTCTCACCGG AGTGGCGATC GCAGAGTTGA TTCCTTTTGA AATTAAGGGC

1381 CTCGAGTTCG CCCTTTGCTC TCTCTTTGTC ACGCTGACTT TGGATTCCTG CCGAACGAAA

1441 AAGCAGATCC CTTCTCTGCT GCTCGCAGGT TTGAGCTTCA CCATTGCTCT TGTGGTAATT

1501 CCAGGTCAGG CCCTATTTGC GGCGCTGCTG ATCTTCTTGG GTCTGTTGAC CATCCGGTAC

1561 TTCTTCTTGG GAAAGGCTGC TAAATGACAA CTGATTTCTC CTGTATTCTC CTTGTTGTCG

1621 CAGTATGTGC AGTCATTACT TTTGCGCTCC GGGCGGTTCC GTTCTTAATC CTTAAGCCCC

1681 TACGTGAATC ACAATTTGTG GGCAAAATGG CGATGTGGAT GCCAGCAGGA ATCCTTGCCA

1741 TTTTGACCGC ATCAACGTTT CGCAGCAATG CGATAGATCT GAAGACTCTA ACCTTTGGTC

1801 TCATTGCCGT TGCGATTACA GTGGTGGCGC ATCTTCTTGG CGGTCGACGC ACCTTGTTGA

1861 GCGTTGGCGC TGGCACCATC GTTTTTGTTG GACTGGTGAA TCTTTTCTAA CTAAAAGCAC
 
1921 TACCTGTGAA GGGATGTCAG GACGTTACAG TTACAGCAAA TGAAACTTAT CGGATTCGCA

1981 CCGGAAGAGA ACAAATCAGC ATCGGAAGGT TTGCTCTAAA TGGAAAGCGT GCAAGCTGGG

2041 TTTGTCATCC AGATGAAACA TTTATTGGTG TACCAAAAGT CATTTCTACA CTACCGGATA

2101 TTCAAAGCAT TGATGTAACG CGTTACACGT GCTGACAAAA CAGCATTACA GCCAGCAGGA

2161 AGTACTGCGT TGGATCGATG TCTGTTCAGG GACTCAACCT AATGCAAAGG ATCCCGCATT

2221 TCTTAAAGTC AGGGCGCATA TCTTCCAGCG TAATACCTAG GGAATATGGG CTTGTGTTGA

2281 TAAAGATTGC AGATTAAAGC ACGGTACACC GCTCGACAAA GGCTGGCCCT TTGGCTATGT

2341 GTATGTGAAC CAGCGACAAA ATTGTGACTG TGGAAGCCCT GTATACGAAG TTGCATTCTG

2401 TAATGATTGT AATGAGCCTC ATCTTCTGGC ACGGGACAAA AAGGGCAAAC TAGTCCAGTG

2461 GGAAAATAAA GGTGGCGATG AATTCTCTTT GCAGGATGAA GTACCTGTTG AACATGACGC

2521 TACAGAAGAA AAAGTCGAAA AAGAGAACAG TTTTCAGCCT CCGTTGATTA TTGCCGCAG
SEQ ID No.2: brnF protein (from Corynebacterium glutamicum ATCC13032) 
1 VQKTQEIHSS LEVSPSKAAL EPDDKGYRRY EIAQGLKTSL AAGLGMYPIG IAFGLLVIQY

61 GYEWWAAPLF SGLIFAGSTE MLVIALVVGA APLGAIALTT LLVNFRHVFY AFSFPLHVVK

121 NPIARFYSVF ALIDEAYAVT AARPAGWSAW RLISMQIAFH SYWVFGGLTG VAIAELIPFE

181 IKGLEFALCS LFVTLTLDSC RTKKQIPSLL LAGLSFTIAL VVIPGQALFA ALLIFLGLLT

241 IRYFFLGKAA K
SEQ ID No.3: brnE protein (from Corynebacterium glutamicum ATCC13032) 
1 MTTDFSCILL VVAVCAVITF ALRAVPFLIL KPLRESQFVG KMAMWMPAGI LAILTASTFR

61 SNAIDLKTLT FGLIAVAITV VAHLLGGRRT LLSVGAGTIV FVGLVNLF
SEQ ID No.4: Homologous recombination DNA fragment △yjiV-Ptrc-ilvE-PilvD-ilvD sequence (4732bp), artificial sequence 
1 TGAATGGACT GCTATGCGCA GCCTCTGTAG AGGCTAAGCC TCGACTACAA AAAAATAAGA

61 GTACGCGTTG CCAATTCTAT TGGTCAGAAA AGCATCCAGA TGAGCTTAGG GTGATAGTAT

121 CTCTTCCGGA CGAAGTTTCC TTTCCTGTAA CAAGCGAGCC GTCAACTACG CGCTTTGAAC

181 TTGCCATTTG TGAAGATGGT GAGGAAGTCT CTGGCCTTGG GCCAGCCTAT GCTTCTCTGG

241 AAAACAGACA GGCAACAGTT CGATTACGTA AAAGCGAAGT GAGATTTGGC AGGCAAAATC

301 CATCGGCAGG TTTGTCGTTA GTGGCTCGTG CTGGAGGGAT GATTGTTGGG AGCATTAAAC

361 TTGATGACAG TGAAATTGCC ATTGGTGAGG TGCCGTTAAC CTTCATCGTT GATGCAGATC

421 AATGGCTGTT ACAGGGACAG GCTTCTTGCA GTGTGCGAAG CAGCGATGTT CTGATTGTGC
 
481 TCCCTCGGGA TAATAGCAAT GTTGCTGGTT TTGATGGCCA ATCGAGGGCA GTAAACGTAT
541 TAGGACTAAA AGCACTACCT GTGAAGGGAT GTCAGGACGT TACAGTTACA GCAAATGAAA
601 CTTATCGGAT TCGCACCGGA AGAGAACAAA TCAGCATCGG AAGGTTTGCT CTAAATGGAA
661 AGCGTGCAAG CTGGGTTTGT CATCCAGATG AAACATTTAT TGGTGTACCA AAAGTCATTT
721 CTACACTACC GGATATTCAA AGCATTGATG TAACGCGTTA CACGTGCTGA CAAAACAGCA
781 TTACAGCCAG CAGGAAGTAC TGCGTTGTTA ACGTCTGCTT AATACACTGT GGCCGTTTTG
841 CAAAAACTGG CTTCTTGAGC GGCGCATGCG TTCAATCCGT TCCTCTGCTA AGCGGTCAGC
901 CGCTAAATAT GCAGGAATGC CGTCACGCTG AGAAATCTCA AGTACACGCT CGATATTGCC
961 GTAAATGCCT TCAACTTTTT TCAATGCACG TTCTGCATTA TAGCCGTAAA GCTCATCTGC
1021 CACGTTGATG ACACCGCCCG CGTTAATCAC GTAATCCGGT GCATAAACGA TGCCCATTTC
1081 GTGAATTTGA TCACCATGGC GTGTCTCTTT TAATTGGTTG TTAGCCGCAC CTGCGATCAC
1141 TTTCGCCTTC AGCTGTTTAA TGGTGTCGTC GTTAATAGTC GCACCAAGGG CACACGGCGC
1201 ATAAATATCG CAGTCTTGTG AATAAATGTC ATCAGGATCT ACCGCACGGG CGCCAAAATC
1261 TTCAACTGCA CGCTGTACAG ATTGTTTGTT GATATCCGTA ACGATTAAGT TTGCTCCTTC
1321 TTCATGCAGG TGGCGGCAAA GGTTATAGGC TACGTTCCCA ACACCCTGTA CAGCAATGGT
1381 TTTTCCTTCA AGAGAGTCGG TTCCGAAAGC AGCTTTAGCT GCTGCCTTCA TTCCTCTGTA
1441 CACCCCGTAC GCTGTGACTG GGGACGGATT TCCAGAAGAG CCGAAAGCAG GAGAAATCCC
1501 TGTGACATAG TCTGTCTCAT CATGAATGAT ATCCATATCC TCGACCGTTG TGCCCACATC
1561 TTCAGCCGTG ATGTATCTGC CATTCAGTCC TTGAATATAG CGGCCAAACG CGCGGAACAT
1621 TTCCTCATTT TTGTCTTTGC GCGGATCGCC GATAATGACT GTTTTTCCGC CGCCAAGGTT
1681 TAAGCCTGCC GCCGCGTTCT TATAGGTCAT GCCTCTTGCC AATCTGAGCG CATCTTCAAT
1741 TGCCGCTTCT TCATTTTCAT ATGTCCACAT TCTCGTTCCG CCAAGCGCCG GACCAAGCGT
1801 TGTATCATGA ATGGCGATAA TCGCTTTTAA TCCAGATTGT TCATCCTGGC AGAATACCAA
1861 TTGTTCGTAA TCGTATTTCT CCATATATTT AAAAAGTTCC ATGGTCTGTT TCCTGTGTGA
1921 AATTGTTATC CGCTCACAAT TCCACACATT ATACGAGCCG GATGATTAAT TGTCAATTAA
1981 CCCCCCAGTT TCGATTTATC GCGCACCGCG CCTTTGTCGG CGCTGGTTGC CAGGCTGGCA
2041 TAAGCACGCA GGGCAAAGGA GACCTGACGT TCACGATTTT TCGGCGTCCA GGCTTTGTCA
2101 CCTCGAGCGT CCTGCGCTTC ACGACGCGCC GCCAGTTCGG CATCGCTTAC CTGTAACTGA
2161 ATGCCACGGT TCGGGATGTC GATAGCGATC AGGTCACCAT CTTCAATCAG GCCAATGCTG
 
2221 CCGCCGCTTG CCGCTTCCGG TGAGACGTGG CCGATGGAAA GACCAGAGGT GCCACCAGAG

2281 AAACGACCGT CGGTGATCAG CGCACAGGCT TTGCCGAGAC CCATTGATTT CAGGAAGCTG

2341 GTTGGGTAGA GCATTTCCTG CATCCCCGGA CCGCCTTTCG GGCCTTCATA GCGAATTACT

2401 ACCACATCTC CGGCGACAAC TTTACCGCCG AGAATCGCTT CTACCGCATC GTCCTGGCTT

2461 TCGTACACTT TCGCCGGGCC GGTGAATTTG AGGATGCTGT CATCGACGCC TGCCGTTTTC

2521 ACGATGCAGC CGTTTTCCGC AAAGTTACCG TAGAGCACCG CCAGGCCGCC GTCTTTGCTG

2581 TAGGCGTGTT CCAGCGAGCG GATACAGCCA TTGGCGCGAT CGTCGTCCAG CGTATCCCAA

2641 CGGCAATCTT GCGAGAATGC CTGTGTGGTA CGAATGCCTG CAGGACCTGC GCGGAACATA

2701 TTTTTTACCG CGTCATCCTG GGTCAGCATA ACGTCGTATT GTTCCAGCGT TTGCGGCAAC

2761 GTCAGGCCAA GTACGTTTTT CACATCACGG TTCAGTAACC CCGCGCGATC CAGTTCGCCG

2821 AGAATACCGA TAACACCACC AGCACGGTGA ACATCTTCCA TATGGTATTT CTGGGTGCTC

2881 GGCGCAACTT TACACAGCTG TGGAACCTTG CGGGAAAGCT TATCGATATC ACTCATGGTG

2941 AAGTCGATTT CCGCTTCCTG CGCCGCCGCC AGCAGGTGAA GTACGGTGTT AGTCGATCCA

3001 CCCATCGCGA TATCCAGCGT CATGGCGTTT TCAAACGCCG CCTTACTGGC GATATTACGC

3061 GGCAGTGCAC TTTCGTCGTT TTGCTCGTAA TAACGTTTGG TCAATTCAAC AATGCGTTTA

3121 CCAGCATTAA GGAACAGCTG CTTACGGTCG GCGTGGGTTG CCAGCAGCGA GCCGTTGCCC

3181 GGCTGCGACA GGCCCAGCGC TTCGGTCAGG CAGTTCATTG AGTTAGCGGT AAACATCCCG

3241 GAGCAGGAAC CGCAGGTCGG ACACGCGGAA CGTTCAACCT GATCGCTCTG GGAGTCAGAT

3301 ACTTTCGGGT CTGCGCCCTG GATCATCGCA TCAACCAGAT CGAGCTTGAT GATCTGATCG

3361 GAAAGTTTGG TTTTCCCGGC CTCCATCGGG CCGCCGGAAA CAAAGATCAC CGGAATATTC

3421 AGGCGCAGGG AAGCCATCAG CATCCCCGGG GTGATTTTGT CGCAGTTAGA GATGCAGACC

3481 ATGGCGTCGG CGCAGTGGGC GTTGACCATA TACTCAACGG AATCAGCGAT CAGTTCGCGA

3541 GATGGCAGTG AATAAAGCAT CCCCCCGTGG CCCATGGCAA TCCCATCATC CACCGCAATG

3601 GTGTTGAACT CTTTGGCAAC GCCGCCAGCC GCTTCAATTT GTTCGGCGAC CAGTTTACCG

3661 AGATCGCGCA GATGGACGTG ACCCGGTACA AATTGGGTGA ACGAGTTCAC AACCGCGATA

3721 ATCGGCTTAC CGAAATCGGC GTCGGTCATT CCGGTGGCGC GCCACAGCGC ACGAGCACCC

3781 GCCATATTAC GACCATGAGT GGTGGTGGCG GAACGGTACT TAGGCATACT TTATTTACTC

3841 CCAGTGTCTG TCTCGTAAAT GGGACGGTGC GTGCCGTCCC ATTTTTTGTA TTTCAACGGA

3901 TGTGCTGGTG GAGGTGATCG CCTCCTGATG ATGAGCCGCT CCCGATGTGG TGTCGGGAGC
 
3961 GGTATTTTCT ATAAAACTTA CCGCTTATTT GAGATATTCA TCGAAAATGT CGAGTAATTC

4021 TTGATGTATA CACGGCCATT CCTGACCTAA ATTGACGGTA CACAAGCCAA TATCGAAGCC

4081 ATTAATTTTA TAACGATGTT TCACTGCGGT ATCTACGTGG GGATATATTA ATAACCCCCC

4141 TATGTTTTCG CCATTTTCAG GCTTTAACGA CCATAAGTAA TTCATCAGTT GATAAAGATT

4201 TTGCGAATGA AATTTTTCTG TTCCCATTCG TCGTGAAAAA ATGCTCTTAT AGTATTTGGC

4261 GTCAACGATA AGTATTTTTT CTGATGAGCG AATGGTGATG TCAGTTTCCA TTCGAGGTAA

4321 CAAATTAAGT GACTGATCCG ATATACTCGA TGCATCCCAT TTTAAATAAG AGCGGGTTGT

4381 GTTTGCAGAC GTTAATTCAC GACGGCAAAA TTCATAAAGA AACTTTTGAT AAAGTAATGA

4441 CATCTCTTTT TCGTTTCTTT CAAAATCATA GAAACGGTAG TGTCCTTTGT TTTGACCTGG

4501 AATAGAATTA TTGACGATGA ATTTGCAGAC ACTGATAACG AATTTATAAT AACGCGTATT

4561 TTTTCCGCCA TTCAGATAGC TGAAATGCTG CGGAGTTAAA TGAAGAGTGC TAATGCCCGG

4621 TAATTTTCTA TAAAGTGAAC GAGCTTCATC TCTGATAGTT GAATTTAACT TTTCATGCTT

4681 AATTAATATG GCTAATGTGC TTTTTATAAT TCGGTTAGCC AGCGTGTCTT CA
SEQ ID No.5: ilvD protein (from Escherichia coli W3110) 
1 MPKYRSATTT HGRNMAGARA LWRATGMTDA DFGKPIIAVV NSFTQFVPGH VHLRDLGKLV

61 AEQIEAAGGV AKEFNTIAVD DGIAMGHGGM LYSLPSRELI ADSVEYMVNA HCADAMVCIS

121 NCDKITPGML MASLRLNIPV IFVSGGPMEA GKTKLSDQII KLDLVDAMIQ GADPKVSDSQ

181 SDQVERSACP TCGSCSGMFT ANSMNCLTEA LGLSQPGNGS LLATHADRKQ LFLNAGKRIV

241 ELTKRYYEQN DESALPRNIA SKAAFENAMT LDIAMGGSTN TVLHLLAAAQ EAEIDFTMSD

301 IDKLSRKVPQ LCKVAPSTQK YHMEDVHRAG GVIGILGELD RAGLLNRDVK NVLGLTLPQT

361 LEQYDVMLTQ DDAVKNMFRA GPAGIRTTQA FSQDCRWDTL DDDRANGCIR SLEHAYSKDG

421 GLAVLYGNFA ENGCIVKTAG VDDSILKFTG PAKVYESQDD AVEAILGGKV VAGDVVVIRY

481 EGPKGGPGMQ EMLYPTSFLK SMGLGKACAL ITDGRFSGGT SGLSIGHVSP EAASGGSIGL

541 IEDGDLIAID IPNRGIQLQV SDAELAARRE AQDARGDKAW TPKNRERQVS FALRAYASLA

601 TSADKGAVRD KSKLGG
SEQ ID No.6: ilvE protein (from Bacillus subtilis subsp. subtilis str. 168) 
1 MELFKYMEKY DYEQLVFCQD EQSGLKAIIA IHDTTLGPAL GGTRMWTYEN EEAAIEDALR

61 LARGMTYKNA AAGLNLGGGK TVIIGDPRKD KNEEMFRAFG RYIQGLNGRY ITAEDVGTTV

121 EDMDIIHDET DYVTGISPAF GSSGNPSPVT AYGVYRGMKA AAKAAFGTDS LEGKTIAVQG
 
181 VGNVAYNLCR HLHEEGANLI VTDINKQSVQ RAVEDFGARA VDPDDIYSQD CDIYAPCALG

241 ATINDDTIKQ LKAKVIAGAA NNQLKETRHG DQIHEMGIVY APDYVINAGG VINVADELYG

301 YNAERALKKV EGIYGNIERV LEISQRDGIP AYLAADRLAE ERIERMRRSR SQFLQNGHSV

361 LSRR
SEQ ID No.7: Homologous recombination DNA fragment △trpR-Ptrc-ilvH (2108 bp), artificial sequence 
1 CCAATCTGGT GAAGAGCAAG TCAAAAACAG AGCAGGCTCA ACTGGCGCGG TATGCTTTCA

61 ACAACCAATG GTGGGATCTT AGCGTTCAGG CAACGATCGC CGGGAAGCTG TGGGATCATC

121 TGGAAGAGCG ATTCCCGCTG GCTTACAACG ATCTTTTCAA ACGCTACACC AGCGGTAAGG

181 AGATCCCGCA AAGCTATGCG ATGGCGATTG CTCGTCAGGA GAGCGCCTGG AATCCGAAAG

241 TGAAATCACC GGTAGGGGCC AGCGGCTTGA TGCAGATTAT GCCTGGTACA GCGACCCATA

301 CGGTGAAGAT GTTCTCTATT CCCGGTTATA GCAGTCCTGG GCAATTGCTG GATCCGGAAA

361 CGAATATCAA CATTGGCACC AGTTACCTGC AATATGTTTA TCAGCAGTTT GGCAATAATC

421 GTATTTTCTC CTCAGCAGCT TATAACGCCG GACCAGGGCG GGTGCGAACC TGGCTTGGCA

481 ACAGCGCCGG GCGTATCGAC GCAGTGGCAT TTGTCGAGAG TATTCCATTC TCCGAGACGC

541 GCGGTTATGT GAAGAACGTG CTGGCTTATG ACGCTTACTA CCGCTATTTC ATGGGGGATA

601 AACCGACGTT GATGAGCGCC ACGGAATGGG GACGTCGTTA CTGATCCGCA CGTTTATGAT

661 ATGCTATCGT ACTCTTTAGC GAGTACAACC GGGGGAGGCA TTTTGCTTCC CCCGCTAACA

721 ATGGCGACAT ATTATGGCCC AACAATCACC CTATTCAGCA TTGACAATTA ATCATCCGGC

781 TCGTATAATG TGTGGAATTG TGAGCGGATA ACAATTTCAC ACAGGAAACA GACCATGCGC

841 CGGATATTAT CAGTCTTACT CGAAAATGAA TCAGACGCGT TATTCCGCGT GATTGGCCTT

901 TTTTCCCAGC GTGGCTACAA CATTGAAAGC CTGACCGTTG CGCCAACCGA CGATCCGACA

961 TTATCGCGTA TGACCATCCA GACCGTGGGC GATGAAAAAG TACTTGAGCA GATCGAAAAG

1021 CAATTACACA AACTGGTCGA TGTCTTGCGC GTGAGTGAGT TGGGGCAGGG CGCGCATGTT

1081 GAGCGGGAAA TCATGCTGGT GAAAATTCAG GCCAGCGGTT ACGGGCGTGA CGAAGTGAAA

1141 CGTAATACGG AAATATTCCG TGGGCAAATT ATCGATGTCA CACCCTCGCT TTATACCGTT

1201 CAATTAGCAG GCACCAGCGG TAAGCTTGAT GCATTTTTAG CATCGATTCG CGATGTGGCG

1261 AAAATTGTGG AGGTTGCTCG CTCTGGTGTG GTCGGACTTT CGCGCGGCGA TAAAATAATG

1321 CGTTGATTTG GTAGGCATGA TAAGACGCGG CAGCGTCGCA TCAGGCGCTT AATACACGGC
 
1381 ATTATGAAAC GGACTCAGCG CCAGGATCAC CGCCTGGTGA TAGACGCTGG CGCGAGTGAG

1441 TTTCCCGGCG GTAAACACGC CGATCGCCCC TTCCTTACGA CCAATCTCAT CAATACCGGT

1501 ATAACGCGAC ATCACGGGAC CAAGCGCCTC ACCTTCACGC ACTTTTTCCA GAATCACCGC

1561 AGGCAACGGC AAAGTAGCCG AACGCGCCTC GCCGCGCTGG CTGGCGTTTT CAATCACCAC

1621 CCAACTGAAA GTGCTGTCAC CATCGATGCC AGCTTCAATC GCCACCCAAA AATCAGCCTC

1681 TGGAAGTAAA CGGCGGGCAT TGGCTACCCG ATTTCGTGCG CCAGCGCGCG TTTCCTCACT

1741 GCCAAAGGGC TGTTCCGGTA CACCGCTCTC GACGGCAACG GATGCAATAT GGCAGGATCC

1801 TTCGCCGAAG ATCTCGTGAA ATGCCTGCAG AATGGCCTGA ATTTTAGCGG GATTGGTGGT

1861 CGCACAGACA ACTTGGTGCA TAATCAGCAT TACTCAGAAA ATTAACGTTA CAGCAGTATA

1921 CGGAAAAAAA GCATGTTACA GGTATACCTA GTCCGCCACG GTGAAACGCA GTGGAACGCC

1981 GAGCGACGTA TTCAGGGCCA GTCTGACAGC CCGCTGACCG CCAAAGGTGA GCAACAGGCG

2041 ATGCAGGTGG CAACCCGTGC CAAAGAGCTT GGCATTACGC ATATCATCAG TAGCGATTTA

2101 GGACGCAC
SEQ ID No.8: Amino acid sequence (163 aa) encoded by the ilvHG14D and S17F genes, artificial sequence 
1 MRRILSVLLE NESDALFRVI GLFSQRGYNI ESLTVAPTDD PTLSRMTIQT VGDEKVLEQI

61 EKQLHKLVDV LRVSELGQGA HVEREIMLVK IQASGYGRDE VKRNTEIFRG QIIDVTPSLY

121 TVQLAGTSGK LDAFLASIRD VAKIVEVARS GVVGLSRGDK IMR
SEQ ID No.9: Sequence of homologous recombination DNA fragment ΔlacIZ-PxylF-DNA polymerase (4524 bp), artificial sequence 
1 CGGTAATAAT CCACAGCAGG TATTTGCGCA GCCCGAGTTT GTCAGAAAGC AGACCAAACA

61 GCGGTTGGAA TAATAGCGAG AACAGAGAAA TAGCGGCAAA AATAATACCC GTATCACTTT

121 TGCTGATATG GTTGATGTCA TGTAGCCAAA TCGGGAAAAA CGGGAAGTAG GCTCCCATGA

181 TAAAAAAGTA AAAGAAAAAG AATAAACCGA ACATCCAAAA GTTTGTGTTT TTTAAATAGT

241 ACATAATGGA TTTCCTTACG CGAAATACGG GCAGACATGG CCTGCCCGGT TATTATTATT

301 TTTGACACCA GACCAACTGG TAATGGTAGC GACCGGCGCT CAGCTGGAAT TCCGCCGATA

361 CTGACGGGCT CCAGGAGTCG TCGCCACCAA TCCCCATATG GAAACCGTCG ATATTCAGCC

421 ATGTGCCTTC TTCCGCGTGC AGCAGATGGC GATGGCTGGT TTCCATCAGT TGCTGTTGAC

481 TGTAGCGGCT GATGTTGAAC TGGAAGTCGC CGCGCCACTG GTGTGGGCCA TAATTCAATT
 
541 CGCGCGTCCC GCAGCGCAGA CCGTTTTCGC TCGGGAAGAC GTACGGGGTA TACATGTCTG

601 ACAATGGCAG ATCCCAGCGG TCAAAACAGG CGGCAGTAAG GCGGTCGGGA TAGTTTTCTT

661 GCGGCCCTAA TCCGAGCCAG TTTACCCGCT CTGCTACCTG TTACGCGAAC GCGAAGTCCG

721 ACTCTAAGAT GTCACGGAGG TTCAAGTTAC CTTTAGCCGG AAGTGCTGGC ATTTTGTCCA

781 ATTGAGACTC GTGCAACTGG TCAGCGAACT GGTCGTAGAA ATCAGCCAGT ACATCACAAG

841 ACTCATATGT GTCAACCATA GTTTCGCGCA CTGCTTTGAA CAGGTTCGCA GCGTCAGCCG

901 GAATGGTACC GAAGGAGTCG TGAATCAGTG CAAAAGATTC GATTCCGTAC TTCTCGTGTG

961 CCCACACTAC AGTCTTACGA AGGTGGCTAC CGTCTTGGCT GTGTACAAAG TTAGGAGCGA

1021 TACCAGACTC CTGTTTGTGT GCATCAATCT CGCTATCTTT GTTGGTGTTA ATGGTAGGCT

1081 GTAAGCGGAA CTGACCGAGG AACATCAGGT TCAAGCGCGT CTGAATAGGC TTCTTGTATT

1141 CCTGCCACAC AGGGAAACCA TCAGGAGTTA CCCAATGCAC AGCGCAACGC TTGCGAAGAA

1201 TCTCTCCAGT CTTCTTATCT TTGACCTCAG CAGCCAGCAG CTTAGCAGCA GACTTAAGCC

1261 AGTTCATTGC TTCAACCGCA GCTACCACCG TCACGCTCAC AGATTCCCAA ATCAGCTTAG

1321 CCATGTATCC AGCAGCCTGA TTCGGCTGAG TGAACATCAG ACCCTTGCCG GAATCAATAG

1381 CTGGCTGAAT GGTATCTTCC AGCACTTGTT GACGGAAGCC GAACTCTTTG GACCCGTAAG

1441 CCAGCGTCAT GACTGAACGC TTAGTCACAC TGCGAGTAAC ACCGTAAGCC AGCCATTGAC

1501 CAGCCAGTGC CTTAGTGCCC AGCTTGACTT TCTCAGAGAT TTCACCAGTG TTCTCATCGG

1561 TCACGGTAAC TACTTCGTTA TCGGTCCCAT TGATTGCGTC TGCTTGTAGA ATCTCGTTGA

1621 CTTTCTTAGC AACAATCCCG TAGATGTCCT GAACGGTTTC ACTAGGAAGC AAGTTAACCG

1681 CGCGACCACC TACCTCATCT CGGAGCATCG CGGAGAAGTG CTGGATGCCA GAGCAAGACC

1741 CGTCAAACGC CAGCGGAAGG GAGCAGTTAT AGCTCAGGCC GTGGTGCTGT ACCCCAGCGT

1801 ACTCAAAGCA GAACGCAAGG AAGCAGAACG GAGAATCTTG CTCAGCCCAC CAAGTGTTCT

1861 CCAGTGGAGA CTTAGCGCAA GCCATGATGT TCTCGTGGTT TTCCTCAATG AACTTGATGC

1921 GCTCAGGGAA CGGAACCTTA TCGACACCCG CACAGTTTGC ACCGTGGATT TTCAGCCAGT

1981 AGTAACCTTC CTTACCGATT GGTTTACCTT TCGCCAGCGT AAGCAGTCCT TTGGTCATAT

2041 CGTTACCTTG CGGGTTGAAC ATTGACACAG CGTAAACACG ACCGCGCCAG TCCATGTTGT

2101 AAGGGAACCA GATGGCCTTA TGGTTAGCAA ACTTATTGGC TTGCTCAAGC ATGAACTCAA

2161 GGCTGATACG GCGAGACTTG CGAGCCTTGT CCTTGCGGTA CACAGCAGCG GCAGCACGTT

2221 TCCACGCGGT GAGAGCCTCA GGATTCATGT CGATGTCTTC CGGTTTCATC GGGAGTTCTT
 
2281 CACGCTCAAT CGCAGGGATG TCCTCGACCG GACAATGCTT CCACTTGGTG ATTACGTTGG

2341 CGACCGCTAG GACTTTCTTG TTGATTTTCC ATGCGGTGTT TTGCGCAATG TTAATCGCTT

2401 TGTACACCTC AGGCATGTAA ACGTCTTCGT AGCGCATCAG TGCTTTCTTA CTGTGAGTAC

2461 GCACCAGCGC CAGAGGACGA CGACCGTTAG CCCAATAGCC ACCACCAGTA ATGCCAGTCC

2521 ACGGCTTAGG AGGAACTACG CAAGGTTGGA ACATCGGAGA GATGCCAGCC AGCGCACCTG

2581 CACGGGTTGC GATAGCCTCA GCGTATTCAG GTGCGAGTTC GATAGTCTCA GAGTCTTGAC

2641 CTACTACGCC AGCATTTTGG CGGTGTAAGC TAACCATTCC GGTTGACTCA ATGAGCATCT

2701 CGATGCAGCG TACTCCTACA TGAATAGAGT CTTCCTTATG CCACGAAGAC CACGCCTCGC

2761 CACCGAGTAG ACCCTTAGAG AGCATGTCAG CCTCGACAAC TTGCATAAAT GCTTTCTTGT

2821 AGACGTGCCC TACGCGCTTG TTGAGTTGTT CCTCAACGTT TTTCTTGAAG TGCTTAGCTT

2881 CAAGGTCACG GATACGACCG AAGCGAGCCT CGTCCTCAAT GGCCCGACCG ATTGCGCTTG

2941 CTACAGCCTG AACGGTTGTA TTGTCAGCAC TGGTTAGGCA AGCCAGAGTG GTCTTAATGG

3001 TGATGTACGC TACGGCTTCC GGCTTGATTT CTTGCAGGAA CTGGAAGGCT GTCGGGCGCT

3061 TGCCGCGCTT AGCTTTCACT TCCTCAAACC AGTCGTTGAT GCGTGCAATC ATCTTAGGGA

3121 GTAGGGTAGT GATGAGAGGC TTGGCGGCAG CGTTATCCGC AACCTCACCA GCTTTAAGTT

3181 GACGCTCAAA CATCTTGCGG AAGCGTGCTT CACCCATCTC GTAAGACTCA TGCTCAAGGG

3241 CCAACTGTTC GCGAGCTAAA CGCTCACCGT AATGGTCAGC CAGAGTGTTG AACGGGATAG

3301 CAGCCAGTTC GATGTCAGAG AAGTCGTTCT TAGCGATGTT AATCGTGTTC ATGGTGTAGG

3361 GCCTTCTGTA GTTAGAGGAC AGTTTTAATA AGTAACAATC ACCGCGATAA ACGTAACCAA

3421 TTTTTAGCAA CTAAACAGGG GAAAACAATT ACAGATTTTT ATCTTTCGAT TACGATTTTT

3481 GGTTTATTTC TTGATTTATG ACCGAGATCT TACTTTTGTT GCGCAATTGT ACTTATTGCA

3541 TTTTTCTCTT CGAGGAATTA CCCAGTTTCA TCATTCCATT TTATTTTGCG AGCGAGCGCA

3601 CACTTGTGAA TTATCTCCGC CGAGACAGAA CTTAATGGGC CCGCTAACAG CGCGATTTGC

3661 TGGTGACCCA ATGCGACCAG ATGCTCCACG CCCAGTCGCG TACCGTCTTC ATGGGAGAAA

3721 ATAATACTGT TGATGGGTGT CTGGTCAGAG ACATCAAGAA ATAACGCCGG AACATTAGTG

3781 CAGGCAGCTT CCACAGCAAT GGCATCCTGG TCATCCAGCG GATAGTTAAT GATCAGCCCA

3841 CTGACGCGTT GCGCGAGAAG ATTGTGCACC GCCGCTTTAC AGGCTTCGAC GCCGCTTCGT

3901 TCTACCATCG ACACCACCAC GCTGGCACCC AGTTGATCGG CGCGAGATTT AATCGCCGCG

3961 ACAATTTGCG ACGGCGCGTG CAGGGCCAGA CTGGAGGTGG CAACGCCAAT CAGCAACGAC
 
4021 TGTTTGCCCG CCAGTTGTTG TGCCACGCGG TTGGGAATGT AATTCAGCTC CGCCATCGCC

4081 GCTTCCACTT TTTCCCGCGT TTTCGCAGAA ACGTGGCTGG CCTGGTTCAC CACGCGGGAA

4141 ACGGTCTGAT AAGAGACACC GGCATACTCT GCGACATCGT ATAACGTTAC TGGTTTCACA

4201 TTCACCACCC TGAATTGACT CTCTTCCGGG CGCTATCATG CCATACCGCG AAAGGTTTTG

4261 CGCCATTCGA TGGTGTCAAC GTAAATGCAT GCCGCTTCGC CTTCCGGCCA CCAGAATAGC

4321 CTGCGATTCA ACCCCTTCTT CGATCTGTTT TGCTACCCGT TGTAGCGCCG GAAGATGCTT

4381 TTCCGCTGCC TGTTCAATGG TCATTGCGCT CGCCATATAC ACCAGATTCA GACAGCCAAT

4441 CACCCGTTGT TCACTGCGCA GCGGTACGGC GATAGAGGCG ATCTTCTCCT CCTGATCCCA

4501 GCCGCGGTAG TTCTGTCCGT AACC
SEQ ID No.10: Amino acid sequence of DNA polymerase (from Escherichia coli BL21) 
1 MNTINIAKND FSDIELAAIP FNTLADHYGE RLAREQLALE HESYEMGEAR FRKMFERQLK

61 AGEVADNAAA KPLITTLLPK MIARINDWFE EVKAKRGKRP TAFQFLQEIK PEAVAYITIK

121 TTLACLTSAD NTTVQAVASA IGRAIEDEAR FGRIRDLEAK HFKKNVEEQL NKRVGHVYKK

181 AFMQVVEADM LSKGLLGGEA WSSWHKEDSI HVGVRCIEML IESTGMVSLH RQNAGVVGQD

241 SETIELAPEY AEAIATRAGA LAGISPMFQP CVVPPKPWTG ITGGGYWANG RRPLALVRTH

301 SKKALMRYED VYMPEVYKAI NIAQNTAWKI NKKVLAVANV ITKWKHCPVE DIPAIEREEL

361 PMKPEDIDMN PEALTAWKRA AAAVYRKDKA RKSRRISLEF MLEQANKFAN HKAIWFPYNM

421 DWRGRVYAVS MFNPQGNDMT KGLLTLAKGK PIGKEGYYWL KIHGANCAGV DKVPFPERIK

481 FIEENHENIM ACAKSPLENT WWAEQDSPFC FLAFCFEYAG VQHHGLSYNC SLPLAFDGSC

541 SGIQHFSAML RDEVGGRAVN LLPSETVQDI YGIVAKKVNE ILQADAINGT DNEVVTVTDE

601 NTGEISEKVK LGTKALAGQW LAYGVTRSVT KRSVMTLAYG SKEFGFRQQV LEDTIQPAID

661 SGKGLMFTQP NQAAGYMAKL IWESVSVTVV AAVEAMNWLK SAAKLLAAEV KDKKTGEILR

721 KRCAVHWVTP DGFPVWQEYK KPIQTRLNLM FLGQFRLQPT INTNKDSEID AHKQESGIAP

781 NFVHSQDGSH LRKTVVWAHE KYGIESFALI HDSFGTIPAD AANLFKAVRE TMVDTYESCD

841 VLADFYDQFA DQLHESQLDK MPALPAKGNL NLRDILESDF AFA
SEQ ID No.11: Sequence of homologous recombination DNA fragment △ycgH-Ptrc-ilvC (3189 bp), artificial sequence 
1 AAATGGAGGG ATAACAGCCA GCGCAGTAGT CAGTGAATTT GGTGGCACCA TCTTTATGAA

61 TGGTGATAAT TCAGTCGAGT CGGGTGGGGC ATATTCAGCG GGACTTTTAA GCCAGGTTAA
 
121 TGATTCTGAA AAGATGGTAA ATAACACCCG TCTTGAAACC ACAGATAAAA CGAACATTGT

181 TACCTCTGGG GAAAATGCAG TAGGTGTTCT TGCATGTTCA AGTCCTGGAG AGTCTCGAAC

241 ATGTGTCGAT GCTGTAGATG ATGAAGTTAG TGATTCTAAC AGTTACGAAG TTATTAGCCG

301 TGCTGATTTA AAAATGAATG GTGGTTCCAT AACAACTAAT GGCATTAATA GCTATGGTGC

361 TTATGCTAAT GGGAAAAAAG CATATATTAA TTTAGATTAT GTGGCACTTG AAACTGTGGC

421 TGATGGAAGT TATGCAGTTG CTATTCGACA AGGTAACATT GATATAAAAA ATAGTTCTAT

481 TACAACAACA GGCACTAAAG CCCCCATTGC AAAAATATAC AATGGTGGAG AGTTATTTTT

541 TTCCAATGTC ACCGCGGTAT CAAAACAAGA TAAAGGAATA TCAATTGATG CATCAAATAT

601 CGATTCTCAA GCCAAAATAG CACTATTAAG TGTTGAACTT TCAAGTGCTT TGGATAGTAT

661 TGATGTTAAC AAAACTACAA CGGATGTAAG TATCCTTAAT CGAAGTATTA TCACACCTGG

721 TAATAATGTT CTGGTTAATA ATACTGGAGG TGACTTAAAC ATAATTTCGT CCGACTCTAT

781 TCTAAATGGA GCGTTAACCC GCAACAGCAA TACGTTTCAT ATCTGTCATA TAGCCGCGCA

841 GTTTCTTACC TACCTGCTCA ATCGCATGGC TGCGAATCGC TTCGTTCACA TCACGCAGTT

901 GCCCGTTATC TACCGCGCCT TCCGGAATAG CTTTACCCAG GTCGCCCGGT TGCAGCTCTG

961 CCATAAACGG TTTCAGCAAC GGCACACAAG CGTAAGAGAA CAGATAGTTA CCGTACTCAG

1021 CGGTATCAGA GATAACCACG TTCATTTCGT ACAGACGCTT ACGGGCGATG GTGTTGGCAA

1081 TCAGCGGCAG CTCGTGCAGT GATTCATAAT ATGCAGACTC TTCAATGATG CCGGAATCGA

1141 CCATGGTTTC GAACGCCAGT TCAACGCCCG CTTTCACCAT CGCAATCATC AGTACGCCTT

1201 TATCGAAGTA CTCCTGCTCG CCGATTTTGC CTTCATACTG CGGCGCGGTT TCAAACGCGG

1261 TTTTGCCGGT CTCTTCACGC CAGGTCAGCA GTTTCTTATC ATCGTTGGCC CAGTCCGCCA

1321 TCATACCGGA AGAGAATTCG CCGGAGATGA TGTCGTCCAT ATGTTTCTGG AACAGGGGTG

1381 CCATGATCTC TTTCAGCTGT TCAGAAAGCG CATAAGCACG CAGTTTCGCC GGGTTAGAGA

1441 GACGGTCCAT CATCAGGGTG ATGCCGCCCT GTTTCAGTGC TTCGGTGATG GTTTCCCAAC

1501 CGAACTGAAT CAGTTTTTCT GCGTATGCTG GATCGGTACC TTCTTCCACC AGCTTGTCGA

1561 AGCACAGCAG AGAGCCAGCC TGCAACATAC CGCACAGGAT GGTTTGCTCG CCCATCAGGT

1621 CAGATTTCAC TTCCGCAACG AAGGACGATT CCAGCACACC CGCACGGTGA CCACCGGTTG

1681 CAGCCGCCCA GGCTTTGGCA ATCGCCATGC CTTCGCCTTT CGGATCGTTT TCCGGGTGAA

1741 CGGCAATCAG CGTCGGTACG CCGAACCCAC GTTTGTACTC TTCACGCACT TCGGTGCCTG

1801 GGCATTTCGG CGCAACCATC ACTACGGTGA TATCTTTACG GATCTGCTCG CCCACTTCGA
 
1861 CGATGTTGAA ACCGTGCGAG TAGCCCAGCG CCGCGCCGTC TTTCATCAGT GGCTGTACGG

1921 TGCGCACTAC ATCAGAGTGC TGCTTGTCCG GCGTCAGGTT AATCACCAGA TCCGCCTGTG

1981 GGATCAGTTC TTCGTAAGTA CCCACTTTAA AACCATTTTC GGTCGCTTTA CGCCAGGACG

2041 CGCGCTTCTC GGCAATCGCT TCTTTACGCA GAGCGTAGGA GATATCGAGA CCAGAATCAC

2101 GCATGTTCAG GCCCTGGTTC AGACCCTGTG CGCCACAGCC GACGATGACT ACTTTTTTAC

2161 CCTGAAGGTA GCTCGCGCCA TCGGCGAATT CATCGCGGCC CATAAAGCGA CATTTGCCCA

2221 GCTGTGCCAG CTGCTGGCGC AGATTCAGTG TATTGAAGTA GTTAGCCATG GTCTGTTTCC

2281 TGTGTGAAAT TGTTATCCGC TCACAATTCC ACACATTATA CGAGCCGGAT GATTAATTGT

2341 CAAAAACTTG GCGTAAATGG CAACCTGAAT CCAGCGGCCA GCGTCTGGGG AATGTGGGCG

2401 TGCAGCTGGG TGATAATGGC TACAATGACA CCGCAGTGAT GGTGGGCCTG AAATATAAGT

2461 TCTGATCCCG CCGTTAGCTA AAAAACCGCG TCGTATTCAT CGACGCGGTA CATATGAAAT

2521 ATTATTTTTT GCCGATAGCA CGCATGGTGT CATCAATTGC CGTGATCAAC AGCATTTGCG

2581 GGTCTTTAGC GCAAACCTGA TTCAGTTTTT CTACCACTTT GGCGCTCAGT TCCGGAGATT

2641 GCTCAATTTT TAAATCACGG CTGGCAACGC TGGCATTACC CATTACCGCA ACAATTTCTG

2701 CAACCTGTGC GCTGTCAGTT TTTGCCATTT CGTTGGCTTC TGCGCAAGTA ATATAGGTTT

2761 CTGACGGCAA ACCGTTTTTA ATATTGTAGT CCTGCGCCCA GGTCATTGGT GCGAAAACAA

2821 ACAGGCCCGC CAGTAAAGCA AATTTTTTCA TCATCATTCC TTATTTCATT TTACCCAGAA

2881 TTGCACCACC CGTACCGCCA ATCACGGCAC CTTTAATCGC CCCTTCGAGG CCATTGCCGG

2941 TCAGAACGCC AGTGACAGCA CCAACGGCGG CACCCACTTT TGCACCTTTA CGCGCATTTT

3001 TACCGTCGCG GCCTTTTTCT GTTACTGCAC CAACACCAGC GCCAACAGCT GCACCTTTCA

3061 GTACGCCATT AACACCATTG CCAGTAAGTA AACCAACGCC TGCGCCTAGC AATGCACCTT

3121 TCGTGGTGCG GTTCATATCC GCCATCGCTG GCGTGGAGCA GAACAATGCT GAGATAAGCC

3181 CGAAGGCAA
SEQ ID No.12: Amino acid sequence of ilvC protein (from Escherichia coli W3110) 
1 MANYFNTLNL RQQLAQLGKC RFMGRDEFAD GASYLQGKKV VIVGCGAQGL NQGLNMRDSG

61 LDISYALRKE AIAEKRASWR KATENGFKVG TYEELIPQAD LVINLTPDKQ HSDVVRTVQP

121 LMKDGAALGY SHGFNIVEVG EQIRKDITVV MVAPKCPGTE VREEYKRGFG VPTLIAVHPE

181 NDPKGEGMAI AKAWAAATGG HRAGVLESSF VAEVKSDLMG EQTILCGMLQ AGSLLCFDKL

241 VEEGTDPAYA EKLIQFGWET ITEALKQGGI TLMMDRLSNP AKLRAYALSE QLKEIMAPLF
 
301 QKHMDDIISG EFSSGMMADW ANDDKKLLTW REETGKTAFE TAPQYEGKIG EQEYFDKGVL

361 MIAMVKAGVE LAFETMVDSG IIEESAYYES LHELPLIANT IARKRLYEMN VVISDTAEYG

421 NYLFSYACVP LLKPFMAELQ PGDLGKAIPE GAVDNGQLRD VNEAIRSHAI EQVGKKLRGY

481 MTDMKRIAVA G
SEQ ID No.13: Sequence of homologous recombination DNA fragment Δ yjiV-Ptrc-ilvE-PilvD-ilvD-2 (4713 bp), artificial sequence

1 AGTTCGCCCT TTGCTCTCTC TTTGTCACGC TGACTTTGGA TTCCTGCCGA ACGAAAAAGC

61 AGATCCCTTC TCTGCTGCTC GCAGGTTTGA GCTTCACCAT TGCTCTTGTG GTAATTCCAG

121 GTCAGGCCCT ATTTGCGGCG CTGCTGATCT TCTTGGGTCT GTTGACCATC CGGTACTTCT

181 TCTTGGGAAA GGCTGCTAAA TGACAACTGA TTTCTCCTGT ATTCTCCTTG TTGTCGCAGT

241 ATGTGCAGTC ATTACTTTTG CGCTCCGGGC GGTTCCGTTC TTAATCCTTA AGCCCCTACG

301 TGAATCACAA TTTGTGGGCA AAATGGCGAT GTGGATGCCA GCAGGAATCC TTGCCATTTT

361 GACCGCATCA ACGTTTCGCA GCAATGCGAT AGATCTGAAG ACTCTAACCT TTGGTCTCAT

421 TGCCGTTGCG ATTACAGTGG TGGCGCATCT TCTTGGCGGT CGACGCACCT TGTTGAGCGT

481 TGGCGCTGGC ACCATCGTTT TTGTTGGACT GGTGAATCTT TTCTAACTAA AAGCACTACC

541 TGTGAAGGGA TGTCAGGACG TTACAGTTAC AGCAAATGAA ACTTATCGGA TTCGCACCGG

601 AAGAGAACAA ATCAGCATCG GAAGGTTTGC TCTAAATGGA AAGCGTGCAA GCTGGGTTTG

661 TCATCCAGAT GAAACATTTA TTGGTGTACC AAAAGTCATT TCTACACTAC CGGATATTCA

721 AAGCATTGAT GTAACGCGTT ACACGTGCTG ACAAAACAGC ATTACAGCCA GCAGGAAGTA

781 CTGCGTTGTT AACGTCTGCT TAATACACTG TGGCCGTTTT GCAAAAACTG GCTTCTTGAG

841 CGGCGCATGC GTTCAATCCG TTCCTCTGCT AAGCGGTCAG CCGCTAAATA TGCAGGAATG

901 CCGTCACGCT GAGAAATCTC AAGTACACGC TCGATATTGC CGTAAATGCC TTCAACTTTT

961 TTCAATGCAC GTTCTGCATT ATAGCCGTAA AGCTCATCTG CCACGTTGAT GACACCGCCC

1021 GCGTTAATCA CGTAATCCGG TGCATAAACG ATGCCCATTT CGTGAATTTG ATCACCATGG

1081 CGTGTCTCTT TTAATTGGTT GTTAGCCGCA CCTGCGATCA CTTTCGCCTT CAGCTGTTTA

1141 ATGGTGTCGT CGTTAATAGT CGCACCAAGG GCACACGGCG CATAAATATC GCAGTCTTGT

1201 GAATAAATGT CATCAGGATC TACCGCACGG GCGCCAAAAT CTTCAACTGC ACGCTGTACA

1261 GATTGTTTGT TGATATCCGT AACGATTAAG TTTGCTCCTT CTTCATGCAG GTGGCGGCAA

1321 AGGTTATAGG CTACGTTCCC AACACCCTGT ACAGCAATGG TTTTTCCTTC AAGAGAGTCG

1381 GTTCCGAAAG CAGCTTTAGC TGCTGCCTTC ATTCCTCTGT ACACCCCGTA CGCTGTGACT

1441 GGGGACGGAT TTCCAGAAGA GCCGAAAGCA GGAGAAATCC CTGTGACATA GTCTGTCTCA

1501 TCATGAATGA TATCCATATC CTCGACCGTT GTGCCCACAT CTTCAGCCGT GATGTATCTG

1561 CCATTCAGTC CTTGAATATA GCGGCCAAAC GCGCGGAACA TTTCCTCATT TTGTCTTTG

1621 CGCGGATCGC CGATAATGAC TGTTTTTCCG CCGCCAAGGT TTAAGCCTGC CGCCGCGTTC

1681 TTATAGGTCA TGCCTCTTGC CAATCTGAGC GCATCTTCAA TTGCCGCTTC TTCATTTTCA

1741 TATGTCCACA TTCTCGTTCC GCCAAGCGCC GGACCAAGCG TTGTATCATG AATGGCGATA

1801 ATCGCTTTTA ATCCAGATTG TTCATCCTGG CAGAATACCA ATTGTTCGTA ATCGTATTTC

1861 TCCATATATT TAAAAAGTTC CATGGTCTGT TTCCTGTGTG AAATTGTTAT CCGCTCACAA

1921 TTCCACACAT TATACGAGCC GGATGATTAA TTGTCAATTA ACCCCCCAGT TTCGATTTAT

1981 CGCGCACCGC GCCTTTGTCG GCGCTGGTTG CCAGGCTGGC ATAAGCACGC AGGGCAAAGG

2041 AGACCTGACG TTCACGATTT TTCGGCGTCC AGGCTTTGTC ACCTCGAGCG TCCTGCGCTT

2101 CACGACGCGC CGCCAGTTCG GCATCGCTTA CCTGTAACTG AATGCCACGG TTCGGGATGT

2161 CGATAGCGAT CAGGTCACCA TCTTCAATCA GGCCAATGCT GCCGCCGCTT GCCGCTTCCG

2221 GTGAGACGTG GCCGATGGAA AGACCAGAGG TGCCACCAGA GAAACGACCG TCGGTGATCA

2281 GCGCACAGGC TTTGCCGAGA CCCATTGATT TCAGGAAGCT GGTTGGGTAG AGCATTTCCT

2341 GCATCCCCGG ACCGCCTTTC GGGCCTTCAT AGCGAATTAC TACCACATCT CCGGCGACAA

2401 CTTTACCGCC GAGAATCGCT TCTACCGCAT CGTCCTGGCT TTCGTACACT TTCGCCGGGC

2461 CGGTGAATTT GAGGATGCTG TCATCGACGC CTGCCGTTTT CACGATGCAG CCGTTTTCCG

2521 CAAAGTTACC GTAGAGCACC GCCAGGCCGC CGTCTTTGCT GTAGGCGTGT TCCAGCGAGC

2581 GGATACAGCC ATTGGCGCGA TCGTCGTCCA GCGTATCCCA ACGGCAATCT TGCGAGAATG

2641 CCTGTGTGGT ACGAATGCCT GCAGGACCTG CGCGGAACAT ATTTTTTACC GCGTCATCCT

2701 GGGTCAGCAT AACGTCGTAT TGTTCCAGCG TTTGCGGCAA CGTCAGGCCA AGTACGTTTT

2761 TCACATCACG GTTCAGTAAC CCCGCGCGAT CCAGTTCGCC GAGAATACCG ATAACACCAC

2821 CAGCACGGTG AACATCTTCC ATATGGTATT TCTGGGTGCT CGGCGCAACT TTACACAGCT

2881 GTGGAACCTT GCGGGAAAGC TTATCGATAT CACTCATGGT GAAGTCGATT TCCGCTTCCT

2941 GCGCCGCCGC CAGCAGGTGA AGTACGGTGT TAGTCGATCC ACCCATCGCG ATATCCAGCG

3001 TCATGGCGTT TTCAAACGCC GCCTTACTGG CGATATTACG CGGCAGTGCA CTTTCGTCGT

3061 TTTGCTCGTA ATAACGTTTG GTCAATTCAA CAATGCGTTT ACCAGCATTA AGGAACAGCT

3121 GCTTACGGTC GGCGTGGGTT GCCAGCAGCG AGCCGTTGCC CGGCTGCGAC AGGCCCAGCG
3181 CTTCGGTCAG GCAGTTCATT GAGTTAGCGG TAAACATCCC GGAGCAGGAA CCGCAGGTCG
3241 GACACGCGGA ACGTTCAACC TGATCGCTCT GGGAGTCAGA TACTTTCGGG TCTGCGCCCT
3301 GGATCATCGC ATCAACCAGA TCGAGCTTGA TGATCTGATC GGAAAGTTTG GTTTTCCCGG
3361 CCTCCATCGG GCCGCCGGAA ACAAAGATCA CCGGAATATT CAGGCGCAGG GAAGCCATCA
3421 GCATCCCCGG GGTGATTTTG TCGCAGTTAG AGATGCAGAC CATGGCGTCG GCGCAGTGGG
3481 CGTTGACCAT ATACTCAACG GAATCAGCGA TCAGTTCGCG AGATGGCAGT GAATAAAGCA
3541 TCCCCCCGTG GCCCATGGCA ATCCCATCAT CCACCGCAAT GGTGTTGAAC TCTTTGGCAA
3601 CGCCGCCAGC CGCTTCAATT TGTTCGGCGA CCAGTTTACC GAGATCGCGC AGATGGACGT
3661 GACCCGGTAC AAATTGGGTG AACGAGTTCA CAACCGCGAT AATCGGCTTA CCGAAATCGG
3721 CGTCGGTCAT TCCGGTGGCG CGCCACAGCG CACGAGCACC CGCCATATTA CGACCATGAG
3781 TGGTGGTGGC GGAACGGTAC TTAGGCATAC TTTATTTACT CCCAGTGTCT GTCTCGTAAA
3841 TGGGACGGTG CGTGCCGTCC CATTTTTTGT ATTTCAACGG ATGTGCTGGT GGAGGTGATC
3901 GCCTCCTGAT GATGAGCCGC TCCCGATGTG GTGTCGGGAG CGGTATTTTC TATAAAACTT
3961 ACCGCTTATT TGAGATATTC ATCGAAAATG TCGAGTAATT CTTGATGTAT ACACGGCCAT
4021 TCCTGACCTA AATTGACGGT ACACAAGCCA ATATCGAAGC CATTAATTTT ATAACGATGT
4081 TTCACTGCGG TATCTACGTG GGGATATATT AATAACCCCC CTATGTTTTC GCCATTTTCA
4141 GGCTTTAACG ACCATAAGTA ATTCATCAGT TGATAAAGAT TTTGCGAATG AAATTTTTCT
4201 GTTCCCATTC GTCGTGAAAA AATGCTCTTA TAGTATTTGG CGTCAACGAT AAGTATTTTT
4261 TCTGATGAGC GAATGGTGAT GTCAGTTTCC ATTCGAGGTA ACAAATTAAG TGACTGATCC
4321 GATATACTCG ATGCATCCCA TTTTAAATAA GAGCGGGTTG TGTTTGCAGA CGTTAATTCA
4381 CGACGGCAAA ATTCATAAAG AAACTTTTGA TAAAGTAATG ACATCTCTTT TTCGTTTCTT
4441 TCAAAATCAT AGAAACGGTA GTGTCCTTTG TTTTGACCTG GAATAGAATT ATTGACGATG
4501 AATTTGCAGA CACTGATAAC GAATTTATAA TAACGCGTAT TTTTTCCGCC ATTCAGATAG
4561 CTGAAATGCT GCGGAGTTAA ATGAAGAGTG CTAATGCCCG GTAATTTTCT ATAAAGTGAA
4621 CGAGCTTCAT CTCTGATAGT TGAATTTAAC TTTTCATGCT TAATTAATAT GGCTAATGTG
4681 CTTTTTATAA TTCGGTTAGC CAGCGTGTCT TCA

## Claims

1. An engineering bacterium producing valine, comprising characteristics of B1), B2), B3), B4), or B5);
B1) comprising B11), B12), and B13):
B11) a yjiT gene is not expressed or weakly expressed, or activity of protein encoded by the yjiT gene is reduced or lost;
B12) content of protein encoded by a brnF gene is increased or activity of protein encoded by the brnF gene is enhanced;
B13) content of protein encoded by a brnE gene is increased or activity of the protein encoded by the brnE gene is enhanced;
B2) comprising B21), B22), and B23):
B21) a yjiV gene is not expressed or weakly expressed, or activity of the protein encoded by the yjiV gene is reduced or lost;
B22) content of protein encoded by an ilvE gene is increased or activity of protein encoded by the ilvE gene is enhanced;
B23) content of protein encoded by an ilvD gene is increased or activity of protein encoded by the ilvD gene is enhanced;
B3) comprising B31) and B32):
B31) a trpR gene is not expressed or weakly expressed, or activity of protein encoded by the trpR gene is reduced or lost;
B32) content of protein encoded by an ilvH^{G14D , S17F} genes is increased or activity of protein encoded the ilvH^{G14D , S17F} genes is enhanced; the ilvH^{G14D , S17F} genes are obtained by replacing a glycine codon at position 14 of an ilvH gene with an aspartic acid codon and a serine codon at position 17 with a phenylalanine codon;
B4) comprising B41), B42), and B43):
B41) a lacI gene is not expressed or weakly expressed, or inhibitory activity of protein encoded by the lacI gene is reduced or lost;
B42) a lacZ gene is not expressed or weakly expressed, or inhibitory activity of protein encoded by the lacZ gene is reduced or lost;
B43) content of DNA polymerase is increased or activity of DNA polymerase is enhanced;
B5) comprising B51) and B52):
b51) a ycgH gene is not expressed or weakly expressed, or activity of the protein encoded by the ycgH gene is reduced or lost;
b52) content of the protein encoded by an ilvC gene is increased or activity of protein encoded by the ilvC gene is enhanced;
the engineered bacterium is Escherichia coli.

2. An engineering bacterium producing valine, which is a recombinant bacterium obtained by modifying a recipient bacterium; the modifying the recipient bacterium comprises A1), A2), A3), A4), or A5);
A1) comprising A11), A12), and A13):
A11) knocking out a yjiT gene of the recipient bacterium, inhibiting expression of the yjiT gene, or inhibiting activity of protein encoded by the yjiT gene;
A12) increasing content of protein encoded by a brnF gene in the recipient bacterium or enhancing activity of protein encoded by the brnF gene;
A13) increasing content of protein encoded by a brnE gene in the recipient bacterium or enhancing activity of protein encoded by the brnE gene;
A2) comprising A21), A22), and A23):
A21) knocking out a yjiV gene of the recipient bacterium, inhibiting expression of a yjiV gene, or inhibiting activity of protein encoded by the yjiV gene;
A22) increasing content of protein encoded by an ilvE gene in the recipient bacterium or enhancing activity of protein encoded by the ilvE gene;
A23) increasing content of protein encoded by an ilvD gene in the recipient bacterium or enhancing activity of protein encoded by the ilvD gene;
A3) comprising A31) and A32):
A31) knocking out a trpR gene of the recipient bacterium, inhibiting expression of a trpR gene, or inhibiting activity of protein encoded by the trpR gene;
A32) increasing content of protein encoded by ilvH^{G14D , S17F} genes in the recipient bacterium or enhancing activity of protein encoded by the ilvH^{G14D , S17F} genes; the ilvH^{G14D , S17F} genes are obtained by replacing a glycine codon at position 14 of an ilvH gene with an aspartic acid codon and a serine codon at position 17 with a phenylalanine codon;
A4) comprising A41), A42), and A43):
A41) knocking out a lacI gene of the recipient bacterium, inhibiting expression of the lacI gene, or inhibiting activity of protein encoded by the lacI gene;
A42) knocking out a lacZ gene of the recipient bacterium, inhibiting expression of a lacZ gene, or inhibiting activity of protein encoded by the lacZ gene;
A43) increasing content of DNA polymerase in the recipient bacterium or enhancing activity of DNA polymerase;
A5) comprising A51) and A52):
A51) knocking out a ycgH gene of the recipient bacterium, inhibiting expression of the ycgH gene, or inhibiting activity of protein encoded by the ycgH gene;
A52) increasing content of protein encoded by an ilvC gene in the recipient bacterium or enhancing activity of protein encoded by the ilvC gene;
the recipient bacterium is Escherichia coli.

3. The engineering bacterium according to claim 1 or 2, wherein the brnF gene and the brnE gene are derived from Corynebacterium glutamicum.

4. The engineering bacterium according to claim 3, wherein the brnF gene encodes the protein set forth in SEQ ID No.2 in a sequence listing;
the brnE gene encodes protein set forth in SEQ ID No.3 in the sequence listing.

5. The engineering bacterium according to claim 4, wherein the brnF gene is a DNA molecule shown at positions 832-1587 of SEQ ID No.1 in the sequence listing;
the brnE gene is a DNA molecule shown at positions 1584-1910 of SEQ ID No.1 in the sequence listing.

6. The engineering bacterium according to any one of claims 1-5, wherein the ilvE gene is derived from Bacillus subtilis;
the ilvD gene is derived from Escherichia coli.

7. The engineering bacterium according to claim 6, wherein the ilvE gene encodes protein set forth in SEQ ID No.6 in the sequence listing;
the ilvD gene encodes protein set forth in SEQ ID No.5 in the sequence listing.

8. The engineering bacterium according to claim 7, wherein the ilvE gene is a DNA molecule shown at positions 808-1902 of SEQ ID No.4 in the sequence listing;
the ilvD gene is a DNA molecule shown at positions 1977-3827 of SEQ ID No.4 in the sequence listing.

9. The engineering bacterium according to any one of claims 1-8, wherein the ilvH gene is derived from Escherichia coli.

10. The engineering bacterium according to claim 9, wherein the ilvH^{G14D , S17F} gene encodes protein set forth in SEQ ID No.8 in the sequence listing.

11. The engineering bacterium according to claim 10, wherein the ilvH^{G14D , S17F} gene is a DNA molecule shown at positions 835-1326 of SEQ ID No.7 in the sequence listing.

12. The engineering bacterium according to any one of claims 1-11, wherein the DNA polymerase is derived from Escherichia coli.

13. The engineering bacterium according to claim 12, wherein the DNA polymerase is protein set forth in SEQ ID No.10 in the sequence listing.

14. The engineering bacterium according to claim 13, wherein a coding gene of the DNA polymerase is a DNA molecule shown at positions 701-3352 of SEQ ID No.9 in the sequence listing.

15. The engineering bacterium according to any one of claims 1-14, wherein the ilvC gene is derived from Escherichia coli.

16. The engineering bacterium according to claim 15, wherein the ilvC gene encodes protein set forth in SEQ ID No.12 in the sequence listing.

17. The engineering bacterium according to claim 16, wherein the ilvC gene is a DNA molecule shown at positions 794-2269 of SEQ ID No.11 in the sequence listing.

18. The engineering bacterium according to any one of claims 1, 3-17, wherein the engineering bacterium also comprises any two, any three, any four, or five of characteristics of B1), B2), B3), B4), and B5).

19. The engineering bacterium according to any one of claims 2-17, wherein the modification also comprises any two, any three, any four, or five of a1), A2), A3), A4), and A5).

20. A method for preparing an engineering bacterium for producing valine, comprising: modifying a recipient bacterium according to any one of A1), A2), A3), A4), or A5) described in claims 2-17 to obtain a target engineering bacterium, the recipient bacterium is Escherichia coli.

21. The method according to claim 20, wherein the method also comprises modifying the recipient bacterium with any two, any three, any four, or five of A1), A2), A3), A4), and A5) described in claims 2-17.

22. A method for preparing L-valine, comprising: culturing the engineering bacterium according to any one of claims 1-19 to obtain L-valine.

23. A use of the engineering bacterium according to any one of claims 1-19 in the production of L-valine, or in the preparation of a product for producing L-valine, or in the preparation of food, feed, or pharmaceuticals comprising L-valine.
